(19) Europäisches Patentamt
European Patent Office
Office européen des brevets

(11) EP 2 900 201 B1

(12) **EUROPEAN PATENT SPECIFICATION**

(45) Date of publication and mention of the grant of the patent:
**17.04.2019 Bulletin 2019/16**

(21) Application number: **13756377.1**

(22) Date of filing: **23.08.2013**

(51) Int Cl.:
*A61K 8/26* (2006.01) *A61K 8/28* (2006.01)
*A61Q 15/00* (2006.01) *A61K 8/90* (2006.01)
*A61K 8/06* (2006.01)

(86) International application number:
**PCT/EP2013/067567**

(87) International publication number:
**WO 2014/048648 (03.04.2014 Gazette 2014/14)**

(54) **ANTIPERSPIRANT OIL-IN-WATER EMULSION COMPRISING AN ALUMINIUM AND/OR ZIRCONIUM ANTIPERSPIRANT SALT OR COMPLEX AND A WATER-INSOLUBLE BLOCK FILM-FORMING ETHYLENIC POLYMER**

SCHWEISSHEMMENDE ÖL-IN-WASSER-EMULSION MIT EINEM SCHWEISSHEMMENDEN ALUMINIUM- UND/ODER ZIRCONIUMSALZ ODER -KOMPLEX UND WASSERUNLÖSLICHES FILMBILDENDES ETHYLENISCHES BLOCKCOPOLYMER

ÉMULSION ANTIPERSPIRANTE HUILE-DANS-EAU, COMPRENANT UN SEL OU COMPLEXE ANTIPERSPIRANT D'ALUMINIUM ET/OU DE ZIRCONIUM ET UN POLYMÈRE ÉTHYLÉNIQUE FILMOGÈNE SÉQUENCÉ INSOLUBLE DANS L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2012 FR 1259119**
**11.10.2012 US 201261712346 P**

(43) Date of publication of application:
**05.08.2015 Bulletin 2015/32**

(73) Proprietor: **L'Oréal**
**75008 Paris (FR)**

(72) Inventors:
- **AUBRUN, Odile**
**F-92160 Antony (FR)**
- **SEBILLOTTE-ARNAUD, Laurence**
**F-94240 L'hay Les Roses (FR)**
- **JALENQUES, Xavier**
**F-92230 Gennevilliers (FR)**

(74) Representative: **Brohmi, Karim**
**L'Oréal**
**Service DIPI**
**9 Rue Pierre Dreyfus**
**92110 Clichy (FR)**

(56) References cited:
**EP-A1- 1 103 249** **EP-A2- 0 824 911**
**EP-A2- 1 374 843** **WO-A1-96/23483**
**WO-A1-2005/030155** **US-A- 5 919 441**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 900 201 B1

## Description

The invention relates to a cosmetic composition in the form of an oil-in-water emulsion comprising at least one aluminium and/or zirconium antiperspirant salt or complex and a water-insoluble block film-forming ethylenic polymer.

The present invention also relates to a cosmetic treatment process for treating human perspiration and, where appropriate, the body odour associated with human perspiration, especially underarm odour, consisting in applying to the surface of the skin to be treated at least one composition as defined previously.

In the cosmetics field, it is known practice to use in topical application, as antiperspirant active agents, astringent salts such as aluminium and/or zirconium salts, which have the effect of limiting or even preventing the flow of sweat. These products are generally available in the form of roll-ons, sticks, aerosols or sprays. Metal salts of this type are effective as antiperspirant active agents, but their efficacy may be further improved.

Antiperspirant roll-ons in the form of an oil-in-water emulsion with an aqueous continuous phase are particularly sought for their novel cosmetic qualities as regards the sensation of freshness on the skin after application, their rapid drying time and their efficacy. However, their efficacy is still not fully satisfactory.

Polymers with antiperspirant properties are known in the prior art.

Patent application WO 93/24105 discloses the use, as antiperspirant active agents, of polymers forming an occlusive film on the skin. The occlusive polymers proposed are of the octylacrylamide/acrylate copolymer or vinyl acetate/butyl maleate/isobornyl acrylate type, alone or in combination with a PVP/linear $\alpha$-olefin polymer such as PVP/eicosene. The cited examples of occlusive polymers are acrylate/octylacrylamide copolymers such as the commercial products Dermacryl 79, Dermacryl LT and Versacryl 40; crotonic acid derivatives such as the commercial product Resin 28-29-30, methacrylic acid/vinylpyrrolidone/t-butyl acrylate copolymers such as Luviflex VMS-35; methyl vinyl ether/ethyl monomaleate copolymers such as the commercial product Gantrez ES225. However, these polymers have the drawback of not being sufficiently effective on reducing perspiration. Furthermore, the occlusive nature of the polymers promotes bacterial proliferation.

Patent application WO 95/27473 discloses the use, as antiperspirant active agents, of insoluble cationic polymers whose main chain is hydrocarbon-based and which comprise hydrophobic quaternary ammonium side groups.

Patent application WO 01/54658 describes anhydrous compositions containing a cyanoacrylate monomer that reacts with sweat to form *in situ* by polymerization a film on the skin that blocks the sweat ducts.

Patent US 6 387 356 describes alcohol-based compositions comprising a cellulose acetate butyrate ester (CAB 553-0.4, CAB 504-0.2) that is capable of forming a thin film on the skin characterized by a certain hardness and water-transporting properties which reduce or eliminate the sensation of moisture associated with perspiration.

Patent application DE 2947060 describes antiperspirant compositions containing a plasticizer-free aqueous dispersion of acrylic resin.

Patent application WO 2011/073 356 describes antiperspirant compositions containing particular film-forming elastomeric polymers.

Patent application FR 2 954 150 describes pressure-sensitive adhesive polymers.

Patent application EP8424911 discloses an aqueous composition comprising antiperspirant actives and water-soluble block polyglycol polymers.

Patent application WO9623483 discloses a microemulsion comprising antiperspirant actives, PEG-7 glyceryl cocoate and cyclomethicone.

Patent US5919441 discloses a base composition comprising a gellant siloxane block polymer, and discloses an oil-in-water emulsion further comprising an aluminium/zirconium antiperspirant active.

Patent application WO2005030155 discloses a photoprotective composition comprising a block polymer having blocks of different glass transition temperature.

Patent applications EP1374843 and EP1103249 disclose the use of a water-insoluble block film-forming ethylenic polymer in structurant systems for anhydrous antiperspirants.

These polymeric antiperspirant systems are still not sufficiently efficient as regards the efficacy towards human perspiration. Furthermore, these polymers are not always compatible with aluminium and/or zirconium salts. They may form a precipitate when they are placed in contact, in particular if they are introduced into an aqueous phase in the presence of aluminium salts. Consequently, the formulations would not be compliant in terms of the stability criterion.

There is thus a need for novel polymers, that can be formulated in formulations in the form of an oil-in-water emulsion based on aluminium and/or zirconium salts, which do not have the drawbacks stated previously and which can increase the efficacy of the said aluminium and/or zirconium salts.

The Applicant has discovered, surprisingly, that the use of an oil-in-water emulsion comprising at least one aluminium and/or zirconium antiperspirant salt or complex and particular ethylenic polymers can make it possible to achieve this objective.

One subject of the present invention is thus a composition in the form of an oil-in-water emulsion comprising, in a cosmetically acceptable medium:

a) a continuous aqueous phase and
b) an oily phase dispersed in the said aqueous phase and
c) at least one water-insoluble block film-forming ethylenic polymer, the said polymer comprising a first block with a glass transition temperature (Tg) of greater than or equal to 85°C and a second block with a Tg of less than or equal to 20°C and
d) at least one aluminium and/or zirconium antiperspirant salt or complex;

wherein said first block with a Tg of greater than or equal to 85°C comprises at least one acrylate monomer of formula $CH_2$=CH-COOR and at least one methacrylate monomer of formula $CH_2=C(CH_3)$-COOR in which R, which may be identical or different, represents a $C_4$ to $C_{12}$ cycloalkyl group and preferably a $C_8$ to $C_{12}$ cycloalkyl; and wherein said second block with a Tg of less than or equal to 20°C comprises at least one monomer chosen, alone or as a mixture, from:

- the acrylates of formula $CH_2=CHCOOR_3$ in which $R_3$ represents a linear or branched $C_1$ to $C_{12}$ unsubstituted alkyl group, with the exception of the tert-butyl group, in which one or more heteroatoms chosen from O, N and S are optionally intercalated;

- the methacrylates of formula $CH_2=C(CH_3)-COOR_4$ in which $R_4$ represents a linear or branched $C_6$ to $C_{12}$ unsubstituted alkyl group, in which one or more heteroatoms chosen from O, N and S are optionally intercalated;

- (meth)acrylic acid; and

- the monomers of formula (I), preferably with x = 1 and Z=COO.

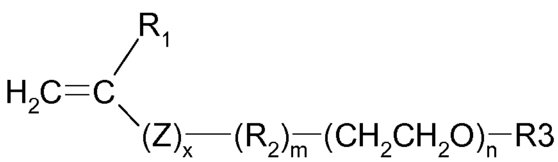

(I)

in which:

- $R_1$ is a hydrogen atom or a methyl radical;

- Z is a divalent group chosen from -COO-, -CONH-, $-CONCH_3-$, -OCO-, -O-, $-SO_2-$, -CO-O-CO- and $-CO-CH_2-CO-$;

- x is 0 or 1;

- $R_2$ is a linear, branched or cyclic, saturated or unsaturated, optionally aromatic divalent carbon-based radical, of 1 to 30 carbon atoms, which may comprise 1 to 18 heteroatoms chosen from O, N, S, F, Si and P;

- m is 0 or 1;

- n is an integer between 3 and 300 inclusive;

- $R_3$ is a hydrogen atom or a linear, branched or cyclic, saturated or unsaturated, optionally aromatic carbon-based radical, of 1 to 30 carbon atoms, which may comprise 1 to 20 heteroatoms chosen from O, N, S, F, Si and P.

The present invention relates to a cosmetic treatment process for treating human perspiration and, where appropriate, the body odour associated with human perspiration, especially underarm odour, consisting in applying to the surface of the skin to be treated at least one composition as defined previously.
The term "aluminium and/or zirconium antiperspirant salt or complex" means any aluminium and/or zirconium antiperspirant salt or complex which has the effect of reducing the flow of sweat and/or of reducing the sensation of moisture associated with human sweat, and/or of masking human sweat.
For the purposes of the present invention, the term "cosmetically acceptable medium" means a medium that is suitable for the topical administration of a composition. A physiologically acceptable medium is preferably a cosmetically or dermatologically acceptable medium, that is to say a medium which is devoid of unpleasant odour or appearance and which is entirely compatible with the topical administration route. In the present case, where the composition is

intended for topical administration, that is to say for administration by application at the surface of the keratinous substance under consideration, such a medium is considered in particular to be physiologically acceptable when it does not cause stinging, tightness or redness unacceptable to the user.

The expression "antiperspirant active agent" means any aluminium salt or complex which, by itself, has the effect of reducing the flow of sweat, of reducing the sensation on the skin of moisture associated with human sweat and of masking human sweat.

The term "oil-in-water emulsion" means a composition comprising a continuous aqueous phase and an oily phase dispersed in the aqueous phase; the two phases being stabilized by an emulsifying system, a thickening system or fillers.

The term "ethylenic polymer" means a polymer obtained by polymerization of ethylenically unsaturated monomers.

The term "film-forming polymer" means any polymer capable of forming, by itself or in the presence of an auxiliary film-forming agent, a continuous film that adheres to a support, in particular to keratin materials such as the skin, the hair, the eyelashes or the nails.

The term "water-insoluble polymer" means that the polymer is not soluble, according to the definition below.

The term "soluble polymer" means that the polymer dissolves in water or in a 50/50 by volume mixture of water and ethanol, or alternatively a mixture of water and isopropanol, without modification of the pH, at a solids content of 5% by weight, at room temperature (25°C, 1 atm.). The polymer is considered to be soluble if it does not form a precipitate or aggregate that is visible to the eye when it is placed in solution, and if it therefore gives a clear solution.

Preferably, the polymer according to the invention is a polymer of linear or grafted structure. In contrast, a polymer of non-linear or ungrafted structure is, for example, a polymer of star or crosslinked structure.

The block ethylenic polymer according to the invention is preferentially prepared exclusively from monofunctional monomers. This means that the block ethylenic polymer does not contain any multifunctional monomers, which make it possible to break the linearity of a polymer so as to obtain in particular a crosslinked polymer, as a function of the content of multifunctional monomer.

Preferably, the polymer according to the invention is a non-elastomeric polymer, i.e. a polymer which, when it is subjected to a constraint intended to stretch it (for example by 30% relative to its initial length), does not return to a length substantially identical to its initial length when the constraint ceases.

More specifically, the term "non-elastomeric polymer" denotes a polymer with an instantaneous recovery $R_i$ < 50% and a delayed recovery $R_{2h}$ < 70% after having been subjected to a 30% elongation. Preferably, $R_i$ is < 30% and $R_{2h}$ is < 50%.

The non-elastomeric nature of the polymer may be determined according to the following protocol: A polymer film is prepared by pouring a solution of the polymer in a Teflon-coated mould, followed by drying for 7 days in an environment conditioned at 23 ± 5°C and 50 ± 10% relative humidity. A film about 100 μm thick is then obtained, from which are cut rectangular specimens (for example using a sample punch) 15 mm wide and 80 mm long. These specimen-shaped samples are subjected to a tensile stress using a machine sold under the reference Zwick, under the same temperature and humidity conditions as for the drying. The specimens are stretched at a speed of 50 mm/min and the distance between the jaws is 50 mm, which corresponds to the initial length (l0) of the specimen.

The instantaneous recovery $R_i$ is determined in the following manner:

- the specimen is stretched by 30% (εmax), i.e. around 0.3 times its initial length (l0);
- the stress is removed by imposing a return speed equal to the tensile speed, i.e. 50 mm/minute, and the residual elongation of the specimen is measured as a percentage, after returning to zero stress ($\varepsilon_i$).

The instantaneous recovery $R_i$ (as a percentage) is given by the formula below:

$$R_i = ((\varepsilon_{max} - \varepsilon_i)/ \varepsilon_{max}) \times 100$$

To determine the delayed recovery, the percentage residual elongation of the specimen ($\varepsilon_{2h}$) is measured 2 hours after returning to zero stress. The delayed recovery $R_{2h}$ (as a percentage) is given by the following formula:

$$R_{2h}= ((\varepsilon_{max} - \varepsilon_{2h})/\varepsilon_{max}) \times 100$$

The polymer according to the present invention is a block polymer, comprising a first block with a Tg of greater than or equal to 85°C and a second block with a Tg of less than or equal to 20°C.

It is pointed out that the terms "first" and "second" blocks do not in any way condition the order of the said blocks in the structure of the polymer.

Preferably, the polymer comprises two distinct blocks (diblock) or, preferentially, three distinct blocks (triblock).

Preferably, the said first and second blocks are mutually incompatible. The term "mutually incompatible blocks" means that the mixture formed from the polymer corresponding to the first block and of the polymer corresponding to the second block is not miscible in the polymerization solvent that is in major amount by weight of the block polymer, at room temperature (25°C) and atmospheric pressure ($10^5$ Pa), for a content of the polymer mixture of greater than or equal to 5% by weight, relative to the total weight of the mixture (polymers and solvent), it being understood that:

i) the said polymers are present in the mixture in a content such that the respective weight ratio ranges from 10/90 to 90/10, and that

ii) each of the polymers corresponding to the first and second blocks has an average (weight-average or number-average) molecular mass equal to that of the block polymer ± 15%.

In the case of a mixture of polymerization solvents, and in the event that two or more solvents are present, the said polymer mixture is immiscible in at least one of them. Needless to say, in the case of a polymerization performed in a single solvent, this solvent is the solvent that is in major amount.

The glass transition temperatures (Tg) indicated are, unless otherwise indicated, theoretical Tg values determined from the theoretical Tg values of the constituent monomers of each of the blocks, which may be found in a reference manual such as the Polymer Handbook, 4th Edition (Brandrup, Immergut, Grulke), 1999, John Wiley, according to the following relationship, known as Fox's law:

$$\frac{1}{Tg} = \sum_i \left(\frac{\varpi i}{Tgi}\right)$$

$w_i$ being the mass fraction of the monomer i in the block under consideration and $Tg_i$ being the glass transition temperature of the homopolymer of the monomer i (expressed in degrees Kelvin).

The polymer according to the invention thus comprises a block with a Tg of greater than or equal to 85°C, for example between 85 and 175°C, preferably between 90 and 150°C and especially between 100 and 130°C.

The polymer according to the invention also comprises a block with a Tg of less than or equal to 20°C, for example between -100 and 20°C, preferably between -80 and 15°C and especially between -60 and 10°C.

Preferably, the block with a Tg of greater than or equal to 85°C represents 50% to 90% by weight and preferably 60% to 80% by weight relative to the weight of the final polymer.

Preferably, the block with a Tg of less than or equal to 20°C represents 5% to 50% by weight and preferably 10% to 40% by weight relative to the weight of the final polymer.

Preferably, the said first and second blocks are linked together via an intermediate segment comprising at least one constituent monomer of the said first block and at least one constituent monomer of the said second block.

The intermediate segment is preferably a block comprising at least one constituent monomer of the first block and at least one constituent monomer of the second block of the polymer, allowing these blocks to be "compatibilized". The said intermediate segment or block is preferably a statistical copolymer.

Preferably, the said intermediate segment or block is derived essentially from constituent monomers of the first block and of the second block.

The term "essentially" means at least 85%, preferably at least 90%, better still 95% and even better still 100%.

Preferably, the said block ethylenic polymer has a polydispersity index Ip of greater than 2, especially between 2 and 9, preferably between 2.3 and 8 and better still between 2.4 and 7. The polydispersity index Ip is equal to the ratio of the weight-average molar mass Mw to the number-average molar mass Mn.

The weight-average molar mass (Mw) and number-average molar mass (Mn) are determined by gel permeation liquid chromatography (THF solvent, calibration curve established with linear polystyrene standards, UV and refractometric detector).

The weight-average molar mass (Mw) of the block ethylenic polymer is preferably between 35 000 and 300 000 and better still between 45 000 and 150 000 g/mol.

The number-average molar mass (Mn) of the block ethylenic polymer is preferably between 10 000 and 70 000 and better still between 12 000 and 50 000 g/mol.

Each block of the polymer according to the invention is derived from one type of monomer or from several different types of monomer. The homopolymer or copolymer of each block of the polymer according to the invention may be statistical, alternating or of another form; preferably statistical. The chemical nature and/or the amount of the monomers constituting each of the blocks may obviously be chosen by a person skilled in the art, on the basis of his general knowledge, to obtain blocks having the required Tg values.

The block with a Tg of greater than or equal to 85°C, or first block, is a copolymer. It preferably comprises at

least one monomer with a Tg of greater than or equal to 85°C.

This block is derived from one or more monomers whose nature and concentration are chosen such that the Tg of the resulting copolymer is greater than or equal to 85°C. The copolymer may comprise, for example, monomers which are such that the homopolymers prepared from these monomers have Tg values of greater than or equal to 85°C, for example a Tg ranging from 85 to 175°C, alone or as a mixture with monomers which are such that the homopolymers prepared from these monomers have Tg values of less than 85°C, preferably chosen from monomers with a Tg of between -100 and 85°C.

Similarly, the block with a Tg of less than or equal to 20°C, or second block, may be a homopolymer or a copolymer. It preferably comprises at least one monomer with a Tg of less than or equal to 20°C.

When this block is a homopolymer, it may be derived from a monomer such that the homopolymer prepared from this monomer has a Tg of less than or equal to 20°C.

When this block is a copolymer, it may be derived from one or more monomers whose nature and concentration are chosen such that the Tg of the resulting copolymer is less than or equal to 20°C. It may comprise, for example, monomers whose corresponding homopolymer has a Tg of less than or equal to 20°C, for example a Tg ranging from -100°C to 20°C, alone or as a mixture with monomers whose corresponding homopolymer has a Tg of greater than 20°C, preferably chosen from monomers with a Tg of between 20 and 175°C.

The block with a Tg of greater than or equal to 85°C comprises at least one acrylate monomer of formula $CH_2=CH-COOR$ and at least one methacrylate monomer of formula $CH_2=C(CH_3)-COOR$ in which R, which may be identical or different, represents a $C_4$ to $C_{12}$ cycloalkyl group and preferably a $C_8$ to $C_{12}$ cycloalkyl; preferably, R is identical in the monomers; preferably, these monomers are isobornyl acrylate and methacrylate.

Most particularly, mention may be made of methyl methacrylate, tert-butyl (meth)acrylate and isobornyl (meth)acrylate, and mixtures thereof.

The block with a Tg of less than or equal to 20°C comprises at least one monomer chosen, alone or as a mixture, from:

- the acrylates of formula $CH_2=CHCOOR_3$ in which $R_3$ represents a linear or branched $C_1$ to $C_{12}$ unsubstituted alkyl group, with the exception of the tert-butyl group, in which one or more heteroatoms chosen from O, N and S are optionally intercalated; especially isobutyl acrylate,
- the methacrylates of formula $CH_2=C(CH_3)-COOR_4$ in which $R_4$ represents a linear or branched $C_6$ to $C_{12}$ unsubstituted alkyl group, in which one or more heteroatoms chosen from O, N and S are optionally intercalated;
- (meth)acrylic acid;
- the monomers of formula (I), preferably with x = 1 and Z=COO.

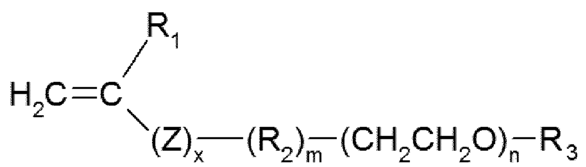

(I)

in which:

- $R_1$ is a hydrogen atom or a methyl radical;
- Z is a divalent group chosen from -COO-, -CONH-, $-CONCH_3-$, -OCO-, -O-, $-SO_2-$, -CO-O-CO- and $-CO-CH_2-CO-$;
- x is 0 or 1;
- $R_2$ is a linear, branched or cyclic, saturated or unsaturated, optionally aromatic divalent carbon-based radical, of 1 to 30 carbon atoms, which may comprise 1 to 18 heteroatoms chosen from O, N, S, F, Si and P;
- m is 0 or 1;
- n is an integer between 3 and 300 inclusive;
- $R_3$ is a hydrogen atom or a linear, branched or cyclic, saturated or unsaturated, optionally aromatic carbon-based radical, of 1 to 30 carbon atoms, which may comprise 1 to 20 heteroatoms chosen from O, N, S, F, Si and P.

Preferably, x = 1 and Z represents COO or CONH, preferentially COO.

In the radical $R_2$, the heteroatom(s), when they are present, may be intercalated in the chain of the said radical $R_2$, or alternatively the said radical $R_2$ may be substituted with one or more groups comprising them such as hydroxyl, amino ($NH_2$, NHR' or NR'R" with R' and R", which may be identical or different, representing a linear or branched $C_1$-$C_{22}$ alkyl, especially methyl or ethyl), $-CF_3$, -CN, $-SO_3H$ or -COOH.

In particular, $R_2$ may comprise a group -O-, -N(R)-, -CO- and a combination thereof, and especially -O-CO-O-, -CO-O-, -N(R)CO-; -O-CO-NR-, -NR-CO-NR-, with R representing H or a linear or branched $C_1$-$C_{22}$ alkyl, optionally comprising 1 to 12 heteroatoms chosen from O, N, S, F, Cl, Br, Si and P.

In particular, $R_2$ may be:

- an alkylene radical containing 1 to 20 carbon atoms, such as methylene, ethylene, n-propylene, isopropylene, n-butylene, isobutylene, tert-butylene, pentylene, isopentylene, n-hexylene, isohexylene, heptylene, isoheptylene, n-octylene, isooctylene, nonylene, isononylene, decylene, isodecylene, n-dodecylene, isododecylene, tridecylene, n-tetradecylene, hexadecylene, n-octadecylene, docosanylene or arachinylene;
- a substituted or unsubstituted cycloalkylene radical containing 5 to 10 carbon atoms, such as cyclopentylene, cyclohexylene, cycloheptylene, cyclooctylene, cyclononylene or cyclodecylene;
- a phenylene radical $-C_6H_4-$ (ortho, meta or para), optionally substituted with a $C_1$-$C_{12}$ alkyl radical optionally comprising 1 to 18 heteroatoms chosen from O, N, S, F, Si and P;
- a benzylene radical $-C_6H_4-CH_2-$ optionally substituted with a $C_1$-$C_{12}$ alkyl radical optionally comprising 1 to 18 heteroatoms chosen from O, N, S, F, Si and P;
- a radical of formula $-CH_2$-O-CO-O-, $CH_2$-$CH_2$-O-CO-O-, $-CH_2$-CO-O-, $-CH_2$-$CH_2$-CO-O-, $-CH_2$-O-CO-NH-, $-CH_2$-$CH_2$-O-CO-NH-; $-CH_2$-NH-CO-NH-, $-CH_2$-$CH_2$-NH-CO-NH-; $-CH_2$-CHOH-, $-CH_2$-$CH_2$-CHOH-, $-CH_2$-$CH_2$-CH($NH_2$)-, $-CH_2$-CH($NH_2$)-, $-CH_2$-$CH_2$-CH(NHR')-, $-CH_2$-CH(NHR')-, $-CH_2$-$CH_2$-CH(NR'R")-, $-CH_2$-CH(NR'R")-, $-CH_2$-$CH_2$-$CH_2$-NR'-, $-CH_2$-$CH_2$-$CH_2$-O-; $-CH_2$-$CH_2$-CHR'-O- with R' and R" representing a linear or branched $C_1$-$C_{22}$ alkyl optionally comprising 1 to 12 heteroatoms chosen from O, N, S, F, Si and P;
- or a mixture of these radicals.

Preferably, $R_2$ may be:

- an alkylene radical containing 1 to 20 carbon atoms, especially methylene, ethylene, n-propylene, n-butylene, n-hexylene, n-octylene, n-dodecylene or n-octadecylene;
- a phenylene radical $-C_6H_4-$ (ortho, meta or para), optionally substituted with a $C_1$-$C_{12}$ alkyl radical optionally comprising 1 to 18 heteroatoms chosen from O, N, S, F, Si and P; or
- a benzylene radical $-C_6H_4-CH_2-$ optionally substituted with a $C_1$-$C_{12}$ alkyl radical optionally comprising 1 to 18 heteroatoms chosen from O, N, S, F, Si and P.

Preferably, n is between 5 and 200 inclusive, better still between 6 and 120 inclusive, or even between 7 and 50 inclusive.

Preferably, $R_3$ is a hydrogen atom; a phenyl radical optionally substituted with a $C_1$-$C_{12}$ alkyl radical optionally comprising 1 to 20 heteroatoms chosen from O, N, S, F, Si and P; a $C_1$-$C_{30}$, especially $C_1$-$C_{22}$ or even $C_2$-$C_{16}$ alkyl radical, optionally comprising 1 to 18 heteroatoms chosen from O, N, S, F, Si and P; a $C_3$-$C_{12}$, especially $C_4$-$C_8$ or even $C_5$-$C_6$ cycloalkyl radical, optionally comprising 1 to 18 heteroatoms chosen from O, N, S, F, Si and P.

Among the radicals $R_3$, mention may be made of methyl, ethyl, propyl, benzyl, ethylhexyl, lauryl, stearyl and behenyl ($-(CH_2)_{21}-CH_3$) chains, and also fluoroalkyl chains, for instance heptadecafluorooctyl sulfonyl amino ethyl $CF_3-(CF_2)_7-SO_2-N(C_2H_5)-CH_2-CH_2$; or alternatively $-CH_2-CH_2-CN$, succinimido, maleimido, mesityl, tosyl, triethoxysilane or phthalimide chains.

Preferentially, the monomers of formula (I) are such that:

- x = 1 and Z represents COO,
- m = 0,
- n = 6 to 120 inclusive,
- $R_3$ is chosen from a hydrogen atom; a phenyl radical optionally substituted with a $C_1$-$C_{12}$ alkyl radical; a $C_1$-$C_{30}$, especially $C_1$-$C_{22}$ or even $C_2$-$C_{16}$ alkyl radical.

Preferably, the monomers of formula (I) have a molecular weight of between 300 and 5000 g/mol.

Among the monomers of formula (I) that are particularly preferred, mention may be made of:

- poly(ethylene glycol) (meth)acrylate in which $R_1$ is H or methyl; Z is COO, x = 1, m=0 and $R_3$ = H;
- methylpoly(ethylene glycol) (meth)acrylate, also known as methoxypoly(ethylene glycol) (meth)acrylate, in which $R_1$ is H or methyl; Z is COO, x = 1, m=0 and $R_3$ = methyl;
- alkylpoly(ethylene glycol) (meth)acrylate in which $R_1$ is H or methyl; Z is COO, x = 1, m=0 and $R_3$ = alkyl;
- phenylpoly(ethylene glycol) (meth)acrylate, also known as poly(ethylene glycol) (meth)acrylate phenyl ether, in which $R_1$ is H or methyl; Z is COO, x = 1, m=0 and $R_3$ = phenyl.

Examples of commercial monomers are:

- CD 350 (methoxypoly(ethylene glycol 350) methacrylate) and CD 550 (methoxypoly(ethylene glycol 550) methacrylate), supplied by Sartomer Chemicals;
- M90G (methoxypoly(ethylene glycol (9 repeating units) methacrylate) and M230G (methoxypolyethylene glycol (23 repeating units) methacrylate) available from Shin-Nakamura Chemicals;
- methoxypoly(ethylene glycol) methacrylates of average molecular weights 300, 475 or 1100, available from Sigma-Aldrich;
- methoxypoly(ethylene glycol) acrylate of average molecular weight 426, available from Sigma-Aldrich;
- the methoxypoly(ethylene glycol) methacrylates available from Laporte under the trade names: MPEG 350, MPEG 550, S10W and S20W, or from Cognis under the name Bisomer;
- poly(ethylene glycol) monomethyl ether mono(succinimidiyl succinate) ester of average molecular weight 1900 or 5000, from Polysciences;
- behenyl poly(ethylene glycol PEG-25) methacrylate, available from Rhodia under the name Sipomer BEM;
- poly(ethylene glycol) phenyl ether acrylates of average molecular weights 236, 280 or 324, available from Aldrich;
- methoxypolyethylene glycol 5000 2-(vinylsulfonyl) ethyl ether commercially available from Fluka;
- polyethylene glycol ethyl ether methacrylate available from Aldrich;
- polyethylene glycol 8000, 4000, 2000 methacrylates from Monomer & Polymer Dajac Laboratories.
- methoxypoly(ethylene glycol) 2000 methacrylate Norsocryl 402 from Arkema;
- methoxypoly(ethylene glycol) 5000 methacrylate Norsocryl 405 from Arkema;
- polyethylene glycol methyl ether acrylate from Aldrich, Mn=454 g/mol, DP = 8-9.

Most particularly, among the monomers with a Tg of less than 20°C, mention may be made of alkyl acrylates in which the alkyl chain comprises from 1 to 10 carbon atoms, with the exception of the tert-butyl group, such as methyl acrylate, isobutyl acrylate and 2-ethylhexyl acrylate; and also poly(ethylene glycol) (meth)acrylates and alkylpoly(ethylene glycol) (meth)acrylates, more particularly methylpoly(ethylene glycol) methacrylates; and mixtures thereof.

The polymer according to the invention may also comprise additional monomers, which may be chosen, alone or as a mixture, from:

- ethylenically unsaturated monomers comprising at least one carboxylic or sulfonic acid function, for instance acrylic acid, methacrylic acid, crotonic acid, maleic anhydride, itaconic acid, fumaric acid, maleic acid, styrenesulfonic acid, acrylamidopropanesulfonic acid, vinylbenzoic acid or vinylphosphoric acid, and salts thereof,
- ethylenically unsaturated monomers comprising at least one hydroxyl function, for instance 2-hydroxypropyl methacrylate, 2-hydroxyethyl methacrylate, 2-hydroxypropyl acrylate or 2-hydroxyethyl acrylate,
- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof.

The block with a Tg of greater than or equal to 85°C comprises at least one acrylate monomer of formula $CH_2$=CH-COOR and at least one methacrylate monomer of formula $CH_2$=C($CH_3$)-COOR in which R, which may be identical or different, represents a $C_4$ to $C_{12}$ cycloalkyl group and preferably a $C_8$ to $C_{12}$ cycloalkyl; preferably, R is identical in the monomers; preferably, these monomers are isobornyl acrylate and methacrylate.

The acrylate monomer and the methacrylate monomer are preferably in mass proportions of between 30/70 and 70/30, preferably between 40/60 and 60/40 and especially of the order of 50/50.

The first block may be obtained exclusively from isobornyl acrylate and methacrylate, which are preferably in an acrylate/methacrylate mass proportion of between 30/70 and 70/30, preferably between 40/60 and 60/40 and especially of about 50/50.

The block with a Tg of less than or equal to 20°C comprises at least one monomer chosen, alone or as a mixture, from:

- the acrylates of formula $CH_2$=$CHCOOR_3$ in which $R_3$ represents a linear or branched $C_1$ to $C_{12}$ unsubstituted alkyl group, with the exception of the tert-butyl group, in which one or more heteroatoms chosen from O, N and S are optionally intercalated; especially isobutyl acrylate,
- the methacrylates of formula $CH_2$=C($CH_3$)-$COOR_4$ in which $R_4$ represents a linear or branched $C_6$ to $C_{12}$ unsubstituted alkyl group, in which one or more heteroatoms chosen from O, N and S are optionally intercalated;
- (meth)acrylic acid;
- the monomers of formula (I), preferably with x = 1 and Z=COO.

Preferentially, the block with a Tg of less than or equal to 20°C comprises acrylic acid and/or methacrylic acid.

The block ethylenic polymer may be obtained by free-radical solution polymerization according to the following preparation process:

- part of the polymerization solvent may be introduced into a suitable reactor and heated until the adequate temperature for the polymerization is reached (typically between 60 and 120°C),
- once this temperature has been reached, the constituent monomers of the first block may be added, in the presence of part of the polymerization initiator,
- after a time T corresponding to a maximum degree of conversion of 90%, preferably, the constituent monomers of the second block and the rest of the initiator may be introduced,
- the mixture may be left to react for a time T' (ranging especially from 3 to 6 hours) after which the mixture is cooled to room temperature (25°C), so as to obtain the polymer dissolved in the polymerization solvent.

The term "polymerization solvent" means a solvent or a mixture of solvents chosen especially from ethyl acetate, butyl acetate, $C_1$-$C_6$ alcohols such as isopropanol or ethanol, and aliphatic alkanes such as isododecane, and mixtures thereof. Preferably, the polymerization solvent is a mixture of butyl acetate and isopropanol or is isododecane.

The polymerization initiator may be chosen from organic peroxides comprising from 8 to 30 carbon atoms. An example that may be mentioned is 2,5-bis(2-ethylhexanoylperoxy)-2,5-dimethylhexane sold under the reference Trigonox® 141 by the company Akzo Nobel.

The block ethylenic polymer according to the invention is preferably prepared by free-radical polymerization and not by controlled or living polymerization. In particular, the polymerization is performed in the absence of control agents, and in particular in the absence of control agents conventionally used in living or controlled polymerization processes, such as nitroxides, alkoxyamines, dithioesters, dithiocarbamates, dithiocarbonates or xanthates, trithiocarbonates or copper-based catalysts, for example.

When it is present, the intermediate segment, or intermediate block, which connects the first block and the second block of the block polymer may result from the polymerization of at least one monomer of the first block, which remains available after the polymerization to a maximum degree of conversion of 90% to form the first block, and of at least one monomer of the second block, added to the reaction mixture. The formation of the second block is initiated when the monomers of the first block no longer react or are no longer incorporated into the polymer chain either because they are all consumed or because their reactivity no longer allows them to be. Thus, the intermediate segment comprises the available monomers of the first block, resulting from a degree of conversion of these first monomers of less than or equal to 90%, during the introduction of the monomers of the second block during the synthesis of the polymer.

Among the block ethylenic polymers of the invention, use will more preferentially be made of a polymer chosen from:

- a poly(isobornyl acrylate/isobornyl methacrylate/isobutyl acrylate/acrylic acid) polymer
- an isobornyl acrylate/isobornyl methacrylate/PEG methacrylate/acrylic acid statistical polymer and more particularly a poly(isobornyl acrylate/isobornyl methacrylate/isobutyl acrylate/acrylic acid) polymer.

The block ethylenic polymers according to the invention are preferably present in the cosmetic composition at a concentration of from 0.05% to 35% by weight, preferably 0.1% to 20% by weight, preferentially 0.5% to 15% by weight and even better still 1% to 10% by weight, of polymer dry matter (DM), relative to the total weight of the composition.

## ANTIPERSPIRANT ACTIVE AGENTS

The compositions contain at least one aluminium and/or zirconium antiperspirant salt.

They are preferably chosen from aluminium and/or zirconium salts; complexes of zirconium hydroxychloride and of aluminium hydroxychloride with an amino acid, such as those described in patent US-3 792 068, commonly known as "ZAG complexes". Such complexes are generally known under the name ZAG (when the amino acid is glycine). ZAG complexes ordinarily have an Al/Zr ratio ranging from about 1.67 to 12.5 and a metal/Cl ratio ranging from about 0.73 to 1.93. Among these products, mention may be made of aluminium zirconium octachlorohydrex GLY, aluminium zirconium pentachlorohydrex GLY, aluminium zirconium tetrachlorohydrate GLY and aluminium zirconium trichlorohydrate GLY.

Among the aluminium salts that may be mentioned are aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulfate, aluminium zirconium octachlorohydrate, aluminium zirconium pentachlorohydrate, aluminium zirconium tetrachlorohydrate, aluminium zirconium trichlorohydrate and more particularly

the aluminium chlorohydrate in activated or non-activated form sold by the company Reheis under the name Microdry Aluminum Chlorohydrate® or by the company Guilini Chemie under the name Aloxicoll PF 40. Aluminium zirconium salts are for example the product sold by the company Reheis under the name Reach AZP-908-SUF®, "activated" aluminium salts, for example the product sold by the company Reheis under the name Reach 103 or by the company Westwood under the name Westchlor 200.

The active agents, antiperspirant salts or complexes, may be present in the composition according to the invention in a proportion from about 0.5% to 25% by weight relative to the total weight of the composition.

## AQUEOUS PHASE

The aqueous phase of the said compositions contains water and generally other water-soluble or water-miscible solvents. The water-soluble or water-miscible solvents comprise monoalcohols with a short chain, for example of $C_1$-$C_4$, such as ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol, glycerol and 1,3-propanediol will be used more particularly.

According to one particular form of the invention, the compositions according to the invention comprise at least one emulsifying surfactant.

## EMULSIFIERS

As emulsifiers that may be used in the oil-in-water emulsions, examples that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate.

Mention may also be made of fatty alcohol/alkylpolyglycoside emulsifying mixtures as described in patent applications WO 92/06778, WO 95/13863 and WO 98/47610, for instance the commercial products sold by the company SEPPIC under the name Montanov ®.

In order to improve the stability on storage of the composition according to the invention and to broaden the possibilities for preparing formulations with a wide range of viscosities and consistencies, according to a particularly preferred form of the invention, the present composition according to the invention comprises a combination of:

(A) at least a mixture comprising at least one alkylpolyglycoside whose alkyl chain is linear or branched and comprises from 12 to 22 carbon atoms and at least one linear or branched fatty alcohol containing from 12 to 22 carbon atoms;
(B) at least one associative nonionic polyurethane polyether.

## Alkylpolyglycoside/fatty alcohol mixtures

The alkylpolyglycosides may be used alone or in the form of mixtures of several alkylpolyglycosides. They generally correspond to the following structure:

$$R(O)(G)x$$

in which the radical R is a linear or branched $C_{12}$-$C_{22}$ alkyl radical, G is a saccharide residue and x ranges from 1 to 5, preferably from 1.05 to 2.5 and more preferentially from 1.1 to 2.

The saccharide residue may be chosen from glucose, dextrose, saccharose, fructose, galactose, maltose, maltotriose, lactose, cellobiose, mannose, ribose, dextran, talose, allose, xylose, levoglucan, cellulose and starch. More preferentially, the saccharide residue denotes glucose.

It should also be noted that each unit of the polysaccharide part of the alkylpolyglycoside may be in $\alpha$ or $\beta$ isomer form, in L or D form, and the configuration of the saccharide residue may be of furanoside or pyranoside type.

It is, of course, possible to use mixtures of alkylpolysaccharides, which may differ from each other in the nature of the borne alkyl unit and/or the nature of the bearing polysaccharide chain.

As regards the fatty alcohols that are to be used, alone or as mixtures, in combination with the alkylpolysaccharides in the emulsifying mixtures in accordance with the invention, they may be linear or branched fatty alcohols, of synthetic origin, or of natural origin, for instance alcohols derived from plant matter (coconut, palm kernel, palm, etc.) or animal matter (tallow, etc.). Needless to say, other long-chain alcohols may also be used, for instance ether alcohols or Guerbet alcohols. Finally, use may also be made of certain more or less long fractions of alcohols of natural origin, for instance coconut ($C_{12}$ to $C_{16}$) or tallow ($C_{16}$ to $C_{18}$) or compounds of diol or cholesterol type.

According to a preferred embodiment of the present invention, the fatty alcohol(s) used are chosen from those containing from 12 to 22 carbon atoms and even more preferentially from 12 to 18 carbon atoms.

As particular examples of fatty alcohols that may be used in the context of the present invention, mention may be made especially of lauryl alcohol, cetyl alcohol, myristyl alcohol, stearyl alcohol, isostearyl alcohol, palmityl alcohol, oleyl alcohol, behenyl alcohol and arachidyl alcohol, which may thus be taken alone or as mixtures.

In addition, it is particularly advantageous, according to the present invention, to use together a fatty alcohol and an alkylpolysaccharide whose alkyl part is identical to that of the selected fatty alcohol.

Fatty alcohol/alkylpolyglycoside emulsifying mixtures as defined above are known per se. They are described in patent applications WO 92/06778, WO 95/13863 and WO 98/47610 and prepared according to the preparation processes indicated in those documents.

Among the fatty alcohol/alkylpolyglycoside mixtures that are particularly preferred, mention may be made of the products sold by the company SEPPIC under the name Montanov®, such as the following mixtures:

cetylstearyl alcohol/cocoyl glucoside - Montanov 82®
arachidyl alcohol and behenyl alcohol/arachidyl glucoside - Montanov 802®
myristyl alcohol/myristyl glucoside - Montanov 14®
cetylstearyl alcohol/cetylstearyl glucoside - Montanov 68®
$C_{14}$-$C_{22}$ alcohols/$C_{12}$-$C_{20}$ alkylglucosides - Montanov L®
cocoyl alcohol/cocoyl glucoside - Montanov S®
isostearyl alcohol/isostearyl glucoside - Montanov WO 18®.

The fatty alcohol/alkylpolyglycoside mixture is preferably present in the emulsions in accordance with the invention in concentrations ranging from 0.5% to 15% by weight and more preferentially from 1% to 10% by weight relative to the total weight of the emulsion.

## Nonionic polyurethane polyethers

The nonionic polyurethane polyethers according to the invention generally comprise, in their chain, both hydrophilic blocks, usually of polyoxyethylene nature, and hydrophobic blocks that may be aliphatic sequences alone and/or cycloaliphatic and/or aromatic sequences.

Preferably, these polyurethane polyethers comprise at least two lipophilic hydrocarbon-based chains containing from 6 to 30 carbon atoms, separated by a hydrophilic block, the hydrocarbon-based chains possibly being pendent chains or chains at the end of the hydrophilic block. In particular, it is possible for one or more pendent chains to be provided. In addition, the polymer may comprise a hydrocarbon-based chain at one end or at both ends of a hydrophilic block.

The polyurethane polyethers may be multiblock, in particular in triblock form. The hydrophobic blocks may be at each end of the chain (for example: triblock copolymer containing a hydrophilic central block) or distributed both at the ends and in the chain (for example multiblock copolymer). These same polymers may also be graft polymers or star polymers.

The nonionic polyurethane polyethers comprising a fatty chain may be triblock copolymers, the hydrophilic block of which is a polyoxyethylene chain comprising from 50 to 1000 oxyethylene groups.

The nonionic polyurethane polyethers comprise a urethane bond between the hydrophilic blocks, whence arises the name.

By extension, also included among the nonionic polyurethane polyethers comprising a hydrophobic chain are those in which the hydrophilic blocks are linked to the hydrophobic blocks via other chemical bonds.

As examples of nonionic polyurethane polyethers comprising a hydrophobic chain that may be used in the invention, it is also possible to use Rheolate 205® containing a urea functional group, sold by the company Rheox, or Rheolate® 208, 204 or 212, and also Acrysol RM 184®.

Mention may also be made of the product Elfacos T210® containing a C12-C14 alkyl chain, and the product Elfacos T212® containing a C18 alkyl chain, from Akzo.

The product DW 1206B® from Röhm & Haas containing a $C_{20}$ alkyl chain and a urethane linkage, sold at a solids content of 20% in water, may also be used.

It is also possible to use solutions or dispersions of these polymers, in particular in water or in aqueous-alcoholic medium. Examples of such polymers that may be mentioned are Rheolate® 255, Rheolate® 278 and Rheolate® 244 sold by the company Rheox. The products DW 1206F and DW 1206J sold by the company Röhm & Haas may also be used.

The polyurethane polyethers that may be used according to the invention may also be chosen from those described in the article by G. Fonnum, J. Bakke and Fk. Hansen - Colloid Polym. Sci., 271, 380-389 (1993).

According to a specific form of the invention, use will be made of a polyurethane polyether that may be obtained

by polycondensation of at least three compounds comprising (i) at least one polyethylene glycol comprising from 150 to 180 mol of ethylene oxide, (ii) a polyoxyethylenated stearyl alcohol comprising 100 mol of ethylene oxide, and (iii) a diisocyanate.
Such polyurethane polyethers are sold especially by the company Sasol Servo BV under the name SER-AD FX 1100®, which is a polycondensate of polyethylene glycol containing 136 mol of ethylene oxide, of stearyl alcohol polyoxyethylenated with 100 mol of ethylene oxide and of hexamethylene diisocyanate (HDI) with a weight-average molecular weight of 30 000 (INCI name: PEG-136/Steareth-100/SMDI Copolymer).

According to another specific form of the invention, use will be made of a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold in particular by the company Röhm & Haas under the names Aculyn 46® and Aculyn 44®.

Aculyn 46® having the INCI name: PEG-150/Stearyl Alcohol/SMDI Copolymer, is a polycondensate of polyethylene glycol comprising 150 or 180 mol of ethylene oxide, of stearyl alcohol and of methyenebis(4-cyclohexyl isocyanate) (SMDI) at 15% by weight in a matrix of maltodextrin (4%) and water (81%)

Aculyn 44® (PEG-150/Decyl Alcohol/SMDI Copolymer) is a polycondensate of polyethylene glycol comprising 150 or 180 mol of ethylene oxide, of decyl alcohol and of methylenebis(4-cyclohexyl isocyanate) (SMDI) at 35% by weight in a mixture of propylene glycol (39%) and water (26%)

The amount of associative polyurethane polyether(s) as active material may range, for example, from 0.1% to 10% by weight, preferably from 0.25% to 8% by weight and better still from 1.5% to 5% by weight relative to the total weight of the composition.

## FATTY PHASE

The compositions according to the invention may contain at least one water-immiscible organic liquid phase, known as a fatty phase. This phase generally comprises one or more hydrophobic compounds that render the said phase water-immiscible. The said phase is liquid (in the absence of a structuring agent) at room temperature (20-25°C). Preferentially, the water-immiscible organic liquid phase in accordance with the invention is generally constituted of at least one volatile oil and/or one non-volatile oil and optionally at least one structuring agent.

The term "oil" means a fatty substance that is liquid at room temperature (25°C) and atmospheric pressure (760 mmHg, i.e. $10^5$ Pa). The oil may be volatile or non-volatile.

For the purposes of the invention, the term "volatile oil" means an oil that is capable of evaporating on contact with the skin or the keratin fibre in less than one hour, at room temperature and atmospheric pressure. The volatile oils of the invention are volatile cosmetic oils, which are liquid at room temperature, having a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at room temperature and atmospheric pressure for at least several hours, and that especially has a vapour pressure of less than $10^{-3}$ mmHg (0.13 Pa).

The oil may be chosen from any physiologically acceptable oil and in particular cosmetically acceptable oil, especially mineral, animal, vegetable or synthetic oils; in particular volatile or non-volatile hydrocarbon oils and/or silicone oils and/or fluoro oils, and mixtures thereof.

More precisely, the term "hydrocarbon oil" means an oil mainly comprising carbon and hydrogen atoms and optionally one or more functional groups chosen from hydroxyl, ester, ether and carboxylic functional groups. Generally, the oil has a viscosity of from 0.5 to 100 000 mPa.s, preferably from 50 to 50 000 mPa.s and more preferably from 100 to 30 000 mPa.s.

As examples of volatile oils that may be used in the invention, mention may be made of:

- volatile hydrocarbon-based oils chosen from hydrocarbon-based oils containing from 8 to 16 carbon atoms, and especially $C_8$-$C_{16}$ isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched $C_8$-$C_{16}$ esters and isohexyl neopentanoate, and mixtures thereof. Other volatile hydrocarbon-based oils, for instance petroleum distillates, especially those sold under the name Shell Solt by the company Shell, may also be used; volatile linear alkanes, such as those described in patent application DE10 2008 012 457 from the company Cognis.
- volatile silicones, for instance volatile linear or cyclic silicone oils, especially those with a viscosity ≤ 8 centistokes ($8\times10^{-6}$ $m^2/s$) and especially containing from 2 to 7 silicon atoms, these silicones optionally comprising alkyl or

alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethyl-hexyltrisiloxane, heptamethyloctyltrisiloxane, hexamethyldisiloxane, octamethyltrisiloxane, decamethyltetrasiloxane or dodecamethylpentasiloxane;
- and mixtures thereof.

Mention may also be made of the volatile linear alkyltrisiloxane oils of general formula (I):

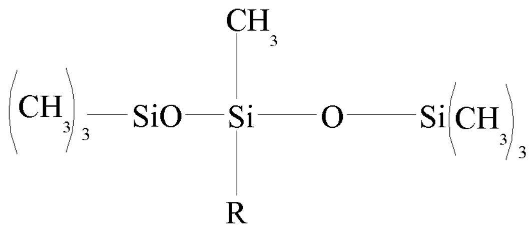

where R represents an alkyl group comprising from 2 to 4 carbon atoms, one or more hydrogen atoms of which can be replaced by a fluorine or chlorine atom.

Mention may be made, among the oils of general formula (I), of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,

corresponding to the oils of formula (I) for which R is, respectively, a butyl group, a propyl group or an ethyl group.

As examples of non-volatile oils that may be used in the invention, mention may be made of:

- hydrocarbon-based oils of animal origin, such as perhydrosqualene;
- hydrocarbon-based plant oils such as liquid triglycerides of fatty acids having 4 to 24 carbon atoms, for instance heptanoic or octanoic acid triglycerides, or else wheatgerm oil, olive oil, sweet almond oil, palm oil, rapeseed oil, cottonseed oil, alfalfa oil, poppyseed oil, pumpkin oil, marrow oil, blackcurrant oil, evening primrose oil, millet oil, barley oil, quinoa oil, rye oil, safflower oil, candlenut oil, passion flower oil, musk rose oil, sunflower oil, corn oil, soybean oil, marrow oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Dynamit Nobel, jojoba oil and shea butter oil;
- linear or branched hydrocarbons, of mineral or synthetic origin, such as liquid paraffins and derivatives thereof, petroleum jelly, polydecenes, polybutenes, hydrogenated polyisobutene such as Parleam, or squalane;

- synthetic ethers containing from 10 to 40 carbon atoms;
- synthetic esters, especially of fatty acids, for instance the oils of formula $R_1COOR_2$ in which $R_1$ represents a linear or branched higher fatty acid residue containing from 1 to 40 carbon atoms and $R_2$ represents a hydrocarbon-based chain, which is especially branched, containing from 1 to 40 carbon atoms, with $R_1 + R_2 \geq 10$, for instance purcellin oil (cetostearyl octanoate), isononyl isononanoate, isopropyl myristate, isopropyl palmitate, $C_{12}$-$C_{15}$ alkyl benzoates, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate;
- fatty alcohols that are liquid at room temperature, containing a branched and/or unsaturated carbon-based chain containing from 12 to 26 carbon atoms, for instance octyldodecanol, isostearyl alcohol, 2-butyloctanol, 2-hexyldecanol, 2-undecylpentadecanol or oleyl alcohol;
- higher fatty acids such as oleic acid, linoleic acid or linolenic acid;
- carbonates;
- acetates;
- citrates;
- fluoro oils that are optionally partially hydrocarbon-based and/or silicone-based, for instance fluorosilicone oils, fluoro

polyethers and fluorosilicones as described in the document EP-A-847 752;
- silicone oils, for instance non-volatile linear or cyclic polydimethylsiloxanes (PDMSs); polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

## STRUCTURING AGENT

The compositions according to the invention comprising a fatty phase may also contain at least one agent for structuring the said fatty phase, which may preferably be chosen from waxes, pasty compounds, and mineral or organic lipophilic gelling agents, and mixtures thereof.

It is understood that the amount of these compounds may be adjusted by a person skilled in the art so as not to harm the effect desired in the context of the present invention.

### Wax(es)

The wax is in general a lipophilic compound that is solid at room temperature (25°C), with a reversible solid/liquid change in state, having a melting point of greater than or equal to 30°C, which may be up to 200°C and in particular up to 120°C.

In particular, the waxes that are suitable for use in the invention may have a melting point of greater than or equal to 45% and in particular of greater than or equal to 55%.

Within the meaning of the invention, the melting point corresponds to the temperature of the most endothermic peak observed in thermal analysis (DSC) as described in Standard ISO 11357-3; 1999. The melting point of the wax may be measured using a differential scanning calorimeter (DSC), for example the calorimeter sold under the name MDSC 2920® by the company TA Instruments.

The measurement protocol is as follows:
A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and it is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions according to the invention are chosen from waxes that are solid at room temperature of animal, vegetable, mineral or synthetic origin, and mixtures thereof.

As illustrations of waxes that are suitable for the invention, mention may be made especially of hydrocarbon-based waxes, for instance beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, refined sunflower wax sold under the name Sunflower Wax® by Koster Keunen, microcrystalline waxes, paraffins and ozokerite; polyethylene waxes, the waxes obtained by Fischer-Tropsch synthesis and waxy copolymers, and also esters thereof.

Mention may also be made of waxes obtained by catalytic hydrogenation of animal or vegetable oils containing linear or branched $C_8$-$C_{32}$ fatty chains. Mention may especially be made, among these waxes, of isomerized jojoba oil such as the trans-isomerized partially hydrogenated jojoba oil manufactured or sold by the company Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate sold under the name Hest 2T-4S® by the company Heterene.

Mention may also be made of silicone waxes ($C_{30-45}$ alkyl dimethicone) and fluoro waxes.

The waxes obtained by hydrogenation of castor oil esterified with cetyl alcohol, sold under the names Phytowax Castor 16L64® and 22L73® by the company Sophim, may also be used. Such waxes are described in patent application FR-A-2 792 190.

A wax that may be used is a $C_{20}$-$C_{40}$ alkyl (hydroxystearyloxy)stearate (the alkyl group containing from 20 to 40 carbon atoms), alone or as a mixture.

Such a wax is especially sold under the names "Kester Wax K 82 P®", "Hydroxypolyester K 82 P®" and "Kester Wax K 80 P®" by the company Koster Keunen.

As microwaxes that may be used in the compositions according to the invention, mention may be made especially

of carnauba microwaxes, such as the product sold under the name MicroCare 350® by the company Micro Powders, synthetic microwaxes, such as the product sold under the name MicroEase 114S® by the company Micro Powders, microwaxes consisting of a mixture of carnauba wax and polyethylene wax, such as the products sold under the names Micro Care 300® and 310® by the company Micro Powders, microwaxes consisting of a mixture of carnauba wax and of synthetic wax, such as the product sold under the name Micro Care 325® by the company Micro Powders, polyethylene microwaxes, such as the products sold under the names Micropoly 200®, 220®, 220L® and 250S® by the company Micro Powders, the commercial products Performalene 400 Polyethylene@ and Performalene 500-L Polyethylene@ from New Phase Technologies, Performalene 655 Polyethylene or paraffin waxes, for instance the wax having the INCI name Microcrystalline Wax® and Synthetic Wax and sold under the trade name Microlease® by the company Sochibo; polytetrafluoroethylene microwaxes such as those sold under the names Microslip 519® and 519 L® by the company Micro Powders.

The composition according to the invention will preferably comprise a content of wax(es) ranging from 3% to 20% by weight relative to the total weight of the composition, in particular from 5% to 15% and more particularly from 6% to 15%.

According to a particular form of the invention, in the context of anhydrous solid compositions in stick form, use will be made of polyethylene microwaxes in the form of crystallites with an aspect ratio at least equal to 2, and with a melting point ranging from 70°C to 110°C and preferably from 70°C to 100°C, in order to reduce or indeed even eliminate the presence of strata in the solid composition.

These crystallites in needle form and especially the dimensions thereof may be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool in order to solidify. Observation of the crystallites is performed using a Leica DMLB100® optical microscope, with an objective lens selected as a function of the size of the objects to be viewed, and under polarized light. The dimensions of the crystallites are measured using image analysis software such as that sold by the company Microvision.

The crystallite polyethylene waxes in accordance with the invention preferably have an average length ranging from 5 to 10 $\mu$m. The term "average length" denotes the dimension given by the statistical particle size distribution at half the population, which is written as D50.

Use will be made more particularly of a mixture of Performalene 400 Polyethylene@ and Performalene 500-L Polyethylene@ waxes from New Phase Technologies.

**Pasty compounds**

Within the meaning of the present invention, the term "pasty compound" is intended to denote a lipophilic fatty compound that undergoes a reversible solid/liquid change in state, which has in the solid form an anisotropic crystal organization, and that comprises, at a temperature of 23°C, a liquid fraction and a solid fraction.

The pasty compound is preferably chosen from synthetic compounds and compounds of vegetable origin. A pasty compound may be obtained by synthesis from starting materials of vegetable origin.

The pasty compound may be advantageously chosen from:

- lanolin and derivatives thereof,
- polymeric or non-polymeric silicone compounds,
- polymeric or non-polymeric fluoro compounds,
- vinyl polymers, especially:
- olefin homopolymers,
- olefin copolymers,
- hydrogenated diene homopolymers and copolymers,
- linear or branched oligomers, homopolymers or copolymers of alkyl (meth)acrylates preferably containing a C8-C30 alkyl group,
- oligomers, which are homopolymers and copolymers of vinyl esters containing C8-C30 alkyl groups, and
- oligomers, which are homopolymers and copolymers of vinyl ethers containing C8-C30 alkyl groups,
- liposoluble polyethers resulting from the polyetherification between one or more C2-C100 and preferably C2-C50 diols,
- esters,
- mixtures thereof.

Among the esters, the following are especially preferred:

- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, especially such as those sold under the brand name Softisan 649® by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched C4-C50 dicarboxylic acid or polycarboxylic acid and a C2-C50 diol or polyol,
- aliphatic esters of an ester, resulting from the esterification of an aliphatic hydroxycarboxylic acid ester with an aliphatic carboxylic acid,
- polyesters resulting from the esterification, with a polycarboxylic acid, of an aliphatic hydroxycarboxylic acid ester, the said ester comprising at least two hydroxyl groups, such as the products Risocast DA-H® and Risocast DA-L®,
- esters of a diol dimer and of a diacid dimer, where appropriate esterified on their free alcohol or acid function(s) with acid or alcohol radicals, such as Plandool-G®,
- mixtures thereof.

Among the pasty compounds of plant origin that will preferably be chosen is a mixture of soybean sterols and of oxyethylenated (5 EO) oxypropylenated (5 PO) pentaerythritol, sold under the reference Lanolide by the company Vevy.

## Lipophilic gelling agents

### Mineral gelling agents

Mineral lipophilic gelling agents that may be mentioned include optionally modified clays, for instance hectorites modified with a C10-C22 ammonium chloride, for instance hectorite modified with distearyldimethylammonium chloride, for instance the product sold under the name Bentone 38V® by the company Elementis.

Mention may also be made of fumed silica optionally subjected to a hydrophobic surface treatment, the particle size of which is less than 1 μm. This is because it is possible to chemically modify the surface of the silica, by chemical reaction generating a reduced number of silanol groups present at the surface of the silica. It is possible especially to substitute silanol groups with hydrophobic groups; a hydrophobic silica is then obtained. The hydrophobic groups may be trimethylsiloxyl groups, which are obtained especially by treating fumed silica in the presence of hexamethyldisilazane. Silicas thus treated are known as "silica silylate" according to the CTFA (8th Edition, 2000). They are sold, for example, under the references Aerosil R812® by the company Degussa, CAB-O-SIL TS-530® by the company Cabot, dimethylsilyloxyl or polydimethylsiloxane groups, which are obtained especially by treating fumed silica in the presence of polydimethylsiloxane or dimethyldichlorosilane. Silicas thus treated are known as "silica dimethyl silylate" according to the CTFA (8th Edition, 2000). They are sold, for example, under the references Aerosil R972® and Aerosil R974® by the company Degussa, and CAB-O-SIL TS-610® and CAB-O-SIL TS-720® by the company Cabot.

The hydrophobic fumed silica in particular has a particle size that may be nanometric to micrometric, for example ranging from about 5 to 200 nm.

### Organic gelling agents

The polymeric organic lipophilic gelling agents are, for example, partially or totally crosslinked elastomeric organopolysiloxanes of three-dimensional structure, for instance those sold under the names KSG6®, KSG16® and KSG18® from Shin-Etsu, Trefil E-505C® or Trefil E-506C® from Dow Corning, Gransil SR-CYC®, SR DMF10®, SR-DC556®, SR 5CYC gel®, SR DMF 10 gel® and SR DC 556 gel® from Grant Industries and SF 1204® and JK 113® from General Electric; ethylcellulose, for instance the product sold under the name Ethocel® by Dow Chemical; galactomannans comprising from one to six and in particular from two to four hydroxyl groups per saccharide, substituted with a saturated or unsaturated alkyl chain, for instance guar gum alkylated with C1 to C6, and in particular C1 to C3, alkyl chains, and mixtures thereof.

Lipophilic gelling agents that may also be mentioned include polymers with a weight-average molecular weight of less than 100 000, comprising a) a polymer backbone with hydrocarbon-based repeating units containing at least one heteroatom, and optionally b) at least one optionally functionalized pendent fatty chain and/or at least one optionally functionalized terminal fatty chain, containing from 6 to 120 carbon atoms and being linked to these hydrocarbon-based units, as described in patent applications WO-A-02/056 847 and WO-A-02/47619, in particular polyamide resins (especially comprising alkyl groups containing from 12 to 22 carbon atoms) such as those described in US-A-5 783 657.

Among the lipophilic gelling agents that may be used in the compositions according to the invention, mention may also be made of fatty acid esters of dextrin, such as dextrin palmitates, especially the products sold under the names Rheopearl TL® or Rheopearl KL® by the company Chiba Flour.

Silicone polyamides of the polyorganosiloxane type such as those described in documents US-A-5 874 069, US-A-5 919 441, US-A-6 051 216 and US-A-5 981 680 may also be used.

These silicone polymers may belong to the following two families:

- polyorganosiloxanes comprising at least two groups capable of establishing hydrogen interactions, these two groups being located in the chain of the polymer, and/or
- polyorganosiloxanes comprising at least two groups capable of establishing hydrogen interactions, these two groups being located on grafts or branches.

**ADDITIVES**

The cosmetic compositions according to the invention may also comprise cosmetic adjuvants chosen from organic powders, deodorant active agents, moisture absorbers, softeners, antioxidants, opacifiers, stabilizers, moisturizers, vitamins, bactericides, preserving agents, polymers, fragrances, thickeners or suspension agents, propellants or any other ingredient usually used in cosmetics for this type of application.

Needless to say, a person skilled in the art will take care to select this or these optional additional compounds such that the advantageous properties intrinsically associated with the cosmetic composition in accordance with the invention are not, or are not substantially, adversely affected by the envisaged addition(s).

**Organic powder**

According to one particular form of the invention, the compositions according to the invention will also contain an organic powder.

In the present application, the term "organic powder" means any solid that is insoluble in the medium at room temperature (25°C).

As organic powders that may be used in the composition of the invention, examples that may be mentioned include polyamide particles and especially those sold under the Orgasol® names by the company Atochem; nylon-6,6 fibres, especially the polyamide fibres sold by Etablissements P Bonte under the name Polyamide 0.9 Dtex 0.3 mm® (INCI name: Nylon-6,6® or Polyamide 6,6) with a mean diameter of 6 μm, a weight of about 0.9 dtex and a length ranging from 0.3 mm to 1.5 mm; polyethylene powders; microspheres based on acrylic copolymers, such as those made of ethylene glycol dimethacrylate/lauryl methacrylate copolymer, sold by the company Dow Corning under the name Polytrap®; polymethyl methacrylate microspheres, sold under the name Microsphere M-100® by the company Matsumoto or under the name Covabead LH85® by the company Wackherr; hollow polymethyl methacrylate microspheres (particle size: 6.5-10.5 μm) sold under the name Ganzpearl GMP 0800® by Ganz Chemical; methyl methacrylate/ethylene glycol dimethacrylate copolymer microbeads (size: 6.5-10.5 μm) sold under the name Ganzpearl GMP 0820® by Ganz Chemical or Microsponge 5640® by the company Amcol Health & Beauty Solutions; ethylene-acrylate copolymer powders, such as those sold under the name Flobeads® by the company Sumitomo Seika Chemicals; expanded powders such as hollow microspheres and especially microspheres formed from a terpolymer of vinylidene chloride, acrylonitrile and methacrylate and sold under the name Expancel® by the company Kemanord Plast under the references 551 DE 12® (particle size of about 12 μm and mass per unit volume of 40 $kg/m^3$), 551 DE 20® (particle size of about 30 μm and mass per unit volume of 65 $kg/m^3$), 551 DE 50® (particle size of about 40 μm), or the microspheres sold under the name Micropearl F 80 ED® by the company Matsumoto; powders of natural organic materials such as starch powders, especially of crosslinked or non-crosslinked corn, wheat or rice starch, such as the powders of starch crosslinked with octenylsuccinic anhydride, sold under the name Dry-Flo® by the company National Starch; silicone resin microbeads such as those sold under the name Tospearl® by the company Toshiba Silicone, especially Tospearl 240®; amino acid powders such as the lauroyllysine powder sold under the name Amihope LL-11® by the company Ajinomoto; particles of wax microdispersion, which preferably have mean sizes of less than 1 μm and especially ranging from 0.02 μm to 1 μm, and which are formed essentially from a wax or a mixture of waxes, such as the products sold under the name Aquacer® by the company Byk Cera, and especially: Aquacer 520 (mixture of synthetic and natural waxes), Aquacer 514 or 513® (polyethylene wax), Aquacer 511 (polymeric wax), or such as the products sold under the name Jonwax 120 by the company Johnson Polymer (mixture of polyethylene wax and paraffin wax) and under the name Ceraflour 961® by the company Byk Cera (micronized modified polyethylene wax); and mixtures thereof.

**Deodorant active agents**

According to one particular form of the invention, the compositions may contain at least one deodorant active agent.

The term "deodorant active agent" means any substance that is capable of reducing, masking or absorbing human body odour and in particular underarm odour.

The deodorant active agents may be bacteriostatic agents or bactericides that act on underarm odour microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol); quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM® from Abitec), glyceryl caprylate or caprate (Dermosoft GMCY® and Dermosoft GMC®, respectively from Straetmans), polyglyceryl-2 caprate (Dermosoft DGMC® from Straetmans), and biguanide derivatives, for instance polyhexamethylene biguanide salts; chlorhexidine and salts thereof; 4-phenyl-4,4-dimethyl-2-butanol (Symdeo MPP® from Symrise); zinc salts such as zinc salicylate, zinc gluconate, zinc pidolate, zinc sulfate, zinc chloride, zinc lactate or zinc phenolsulfonate; salicylic acid and derivatives thereof such as 5-n-octanoylsalicylic acid.

The deodorant active agents may be odour absorbers such as zinc ricinoleates or sodium bicarbonate; metallic or silver or silver-free zeolites, or cyclodextrins and derivatives thereof. They may also be chelating agents such as Dissolvine GL-47-S® from Akzo Nobel, EDTA and DPTA. It may also be a polyol such as glycerol or 1,3-propanediol (Zemea Propanediol sold by Dupont Tate and Lyle BioProducts), or an enzyme inhibitor such as triethyl citrate; or alum.

In the event of incompatibility or to stabilize them, for example, some of the active agents mentioned above may be incorporated into spherules, especially ionic or nonionic vesicles and/or nanoparticles (nanocapsules and/or nanospheres).

The deodorant active agents may also be bacteriostatic agents or bactericidal agents 2,4,4'-trichloro-2'-hydroxydiphenyl ether (Triclosan®), 2,4-dichloro-2'-hydroxydiphenyl ether, 3',4',5'-trichlorosalicylanilide, 1-(3',4'-dichlorophenyl)-3-(4'-chlorophenyl)urea (Triclocarban®) or 3,7,11-trimethyldodeca-2,5,10-trienol (Farnesol®); quaternary ammonium salts such as cetyltrimethylammonium salts or cetylpyridinium salts.

The deodorant active agents may be present in the composition according to the invention in a proportion from about 0.001% to 40% by weight and preferably in a proportion of from about 0.1% to 25% by weight relative to the total composition.

**Moisture absorbers**

It is also possible to add moisture absorbers, for instance perlites and preferably expanded perlites.

The perlites that may be used according to the invention are generally aluminosilicates of volcanic origin and have the composition:

70.0-75.0% by weight of silica $SiO_2$
12.0-15.0% by weight of oxide of aluminium oxide $Al_2O_3$
3.0-5.0% of sodium oxide $Na_2O$
3.0-5.0% of potassium oxide $K_2O$
0.5-2% of iron oxide $Fe_2O_3$ →
0.2-0.7% of magnesium oxide MgO
0.5-1.5% of calcium oxide CaO
0.05-0.15% of titanium oxide $TiO_2$

The perlite is ground, dried and then calibrated in a first step. The product obtained, known as perlite ore, is grey-coloured and has a size of the order of 100 μm.

The perlite ore is subsequently expanded (1000°C/2 seconds) to give more or less white particles. When the temperature reaches 850-900°C, the water trapped in the structure of the material evaporates and brings about the expansion of the material, relative to its original volume. The expanded perlite particles in accordance with the invention may be obtained via the expansion process described in patent US 5 002 698.

Preferably, the perlite particles used will be ground; in this case, they are known as Expanded Milled Perlite (EMP). They preferably have a particle size defined by a median diameter $D_{50}$ ranging from 0.5 to 50 μm and preferably from 0.5 to 40 μm.

Preferably, the perlite particles used have an untamped apparent density at 25°C ranging from 10 to 400 $kg/m^3$ (standard DIN 53468) and preferably from 10 to 300 $kg/m^3$.

Preferably, the expanded perlite particles according to the invention have a water-absorbing capacity, measured at the wet point, ranging from 200% to 1500% and preferably from 250% to 800%.

The wet point corresponds to the amount of water which needs to be added to 1 g of particle in order to obtain a homogeneous paste. This method is derived directly from that of the oil uptake applied to solvents. The measurements are taken in the same way via the wet point and the flow point, which respectively have the following definitions:

wet point: weight, expressed in grams per 100 g of product, corresponding to the production of a homogeneous paste during the addition of a solvent to a powder.

flow point: weight expressed in grams per 100 g of product at and above which the amount of solvent is greater than the capacity of the powder to retain it. This is reflected by the production of a more or less homogeneous mixture that flows over the glass plate.

The wet point and the flow point are measured according to the following protocol:

Protocol for measuring the water absorption

1) Equipment used

Glass plate (25 x 25 mm)
Spatula (wooden shaft and metal part (15 x 2.7 mm))
Silk-bristled brush
Balance

2) Procedure

The glass plate is placed on the balance and 1 g of perlite particles is weighed out. The beaker containing the solvent and the liquid sampling pipette are placed on the balance. The solvent is gradually added to the powder, the whole being regularly blended (every 3 to 4 drops) with the spatula.
The weight of solvent needed to obtain the wet point is noted. Further solvent is added and the weight which makes it possible to reach the flow point is noted. The mean of three tests will be determined.

The expanded perlite particles sold under the trade names Optimat 1430 OR or Optimat 2550 by the company World Minerals will be used in particular.

## Suspension agents

In order to improve the homogeneity of the product, it is also possible to use one or more suspension agents preferably chosen from hydrophobic modified montmorillonite clays such as hydrophobic modified bentonites or hectorites. Examples that may be mentioned include the product Stearalkonium Bentonite (CTFA name) (product of reaction of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (product of reaction of hectorite and distearyldimonium chloride) sold under the name Bentone 38 or Bentone Gel by the company Elementis Specialities.

Other suspension agents may be used, in the present case in hydrophilic (aqueous and/or ethanolic) media. They are cellulose derivatives (which may be microcrystalline), xanthan, guar or starch derivatives (including the modified corn starch sold under the name Structure XL by Akzo Nobel) and locust bean gum or agar derivatives.

The suspension agents are preferably present in amounts ranging from 0.1% to 5% by weight and more preferentially from 0.2% to 2% by weight relative to the total weight of the composition.

The amounts of these various constituents that may be present in the cosmetic composition according to the invention are those conventionally used in compositions for treating perspiration.

## FORMULATION FORMS

The composition according to the invention may be in the form of a more of less thickened cream dispensed in a tube or a grid; in the form of a roll-on (conditioned in ball form); in the form of a solid emulsion stick or in a pressurized form such as a spray or an aerosol device and may in this regard contain the ingredients generally used in products of

this type which are well known to those skilled in the art. Preferably, the composition is in the form of a roll-on.

The compositions according to the invention may also be pressurized and may be packaged in an aerosol device formed by:

(A) a container comprising an antiperspirant composition as defined previously,
(B) at least one propellant and a means for dispensing the said aerosol composition.

The propellants generally used in products of this type and that are well known to those skilled in the art are, for instance, dimethyl ether (DME); volatile hydrocarbons such as n-butane, propane, isobutane and mixtures thereof, optionally with at least one chlorohydrocarbon and/or fluorohydrocarbon; among these derivatives, mention may be made of the compounds sold by the company DuPont de Nemours under the names Freon® and Dymel®, and in particular monofluorotrichloromethane, difluorodichloromethane, tetrafluorodichloroethane and 1,1-difluoroethane sold especially under the trade name Dymel 152 A® by the company DuPont. Carbon dioxide, nitrous oxide, nitrogen or compressed air may also be used as propellant.

The compositions as defined previously and the propellant(s) may be in the same compartment or in different compartments in the aerosol container. According to the invention, the concentration of propellant generally varies from 5% to 95% by weight of pressurized composition, and more preferentially from 50% to 85% by weight relative to the total weight of the pressurized composition.

The dispensing means, which forms a part of the aerosol device, is generally formed by a dispensing valve controlled by a dispensing head, which itself comprises a nozzle via which the aerosol composition is vaporized. The container containing the pressurized composition may be opaque or transparent. It may be made of glass, polymer or metal, optionally coated with a protective varnish coat.

The expressions "between ... and ..." and "ranging from ... to ..." should be understood as meaning limits included, unless otherwise specified.

The examples that follow illustrate the present invention without limiting the scope thereof.

**POLYMER 1: Preparation of a poly(isobornyl acrylate/isobornyl methacrylate/isobutyl acrylate/acrylic acid) polymer**

300 g of isododecane are introduced into a 1-litre reactor and the temperature is then raised so as to pass from 25°C to 90°C over 1 hour. 105 g of isobornyl methacrylate, 105 g of isobornyl acrylate and 1.8 g of 2,5-bis(2-ethylhexanoylperoxy)-2,5-dimethylhexane (Trigonox® 141 from Akzo Nobel) are then added, at 90°C and over 1 hour. The mixture is maintained at 90°C for 1 hour 30 minutes. 75 g of isobutyl acrylate, 15 g of acrylic acid and 1.2 g of 2,5-bis(2-ethylhexanoylperoxy)-2,5-dimethylhexane are then introduced into the preceding mixture, still at 90°C, over 30 minutes. The mixture is maintained at 90°C for 3 hours, and is then cooled.

A solution in isododecane with a dry matter (DM) content of 50% polymer, comprising a first poly(isobornyl acrylate/isobornyl methacrylate) block with a Tg of 128°C, a second poly(isobutyl acrylate/acrylic acid) block with a Tg of -9°C and an intermediate block, which is an isobornyl acrylate/isobornyl methacrylate/isobutyl acrylate/acrylic acid statistical polymer, is obtained.

**POLYMER 2: Preparation of a poly(isobornyl acrylate (35)/isobornyl methacrylate (35)/acrylic acid (5)/PEG methacrylate (25)) polymer**

In a similar manner to Example 1, a solution in isododecane with a dry matter content of 50% polymer, comprising a first poly(isobornyl acrylate/isobornyl methacrylate) block with a Tg of 128°C, a second poly(PEG methacrylate (Bisomer MPEG550)/acrylic acid) block with a Tg of -48°C and an intermediate block, which is an isobornyl acrylate/isobornyl methacrylate/PEG methacrylate/acrylic acid statistical polymer, is obtained.
Mn = 22 600 g/mol; Mw = 56 700 g/mol; Ip = Mw/Mn = 2.5.

The concentration of polymer 1 or 2 in the composition is between 0.5% and 10% and preferably between 0.5% and 6%.

**Example 1: Antiperspirant roll-on**

| Phase | Ingredients | Control formulation | Example 1: (invention) |
|---|---|---|---|
| A | Water | qs 100 | qs 100 |
| | Steareth-100/PEG-136/HDI Copolymer Rheolate FX 1100 (Elementis) | 1 | 1 |
| | Phenoxyethanol Sepicide LD (SEPPIC) | 0.7 | 0.7 |
| B | C14-22 alcohols (and) C12-20 alkyl glucoside Montanov L (SEPPIC) | 3 | 3 |
| | Dimethicone Xiameter PMX-200 Silicone Fluid 350CS (Dow Corning) | 7 | 7 |
| C | Aluminum chlorohydrate Chlorhydrol 50 (Summitreheis) | 30% (15% AM) | 30% (15% AM) |
| D | Perlite | 1 | 1 |
| | Optimat 2550 OR (World Minerals) | | |
| E | Preserving agent | 0.075 | 0.075 |
| F | Polymer 1 | | 10% (5% AM) |

**Procedure**

Phase A is heated to 80°C while stirring with a Rayneri blender.
All of phase C is melted at 80°C in a water bath.
Phase C is introduced into phase A while stirring with a Turrax blender at 80°C for 20 minutes.
The Chlorohydrol 50 is added. The mixture is cooled to 25°C while stirring with a Rayneri blender.
Phase D is added at 35°C.
Phase E is added at 30°C.
Phase F is added slowly at 25°C.

***in vivo* efficacy**

Panel

22 women included, from 21 to 56 years old (average age: 40 years old)
→20 subjects completed the study: 2 subjects withdrew their consent

Synopsis

Study region: the subscapular area of the back
16 areas: Two times 8 areas (4 × 5 $cm^2$) on either side of the spinal column.
8 product areas/8 control areas: 6 test products + 2 references

Each product area has a corresponding symmetrical untreated control area.

4 applications controlled: 1/day
Amount applied on each application: 75 mg
Application method: homogeneous distribution of the antiperspirant products by finger onto the corresponding area by gentle massaging.
Drying time: 15 min

Occlusion for 1 hour. The individuals remain throughout the occlusion period in a room maintained at 30°C and 50% relative humidity.
The antiperspirant efficacy is evaluated 24 hours after the final application.

The back is washed with water to remove any remaining trace of product; cellulose squares are fixed onto the various areas, and the person is then made to sweat in a sauna.
The amount of sweat is evaluated by weighing the cellulose squares before and after sweating.
Measurement conditions:

Sweating in the sauna at 80°C
Sweating time: 15 to 20 mins

Scale of the degree of efficacy:

- percentage efficacy reduction R < 10% no efficacy
- percentage efficacy reduction 10% < R < 15% low efficacy
- percentage efficacy reduction 15% < R < 25% moderate efficacy
- percentage efficacy reduction 25% < R < 35% good efficacy
- percentage efficacy reduction 35% < R < 50% high efficacy
- percentage efficacy reduction R > 50% very high efficacy

The results below show that the polymers described according to the invention are as efficient as the control formulation without polymer.

| **Formulation** | **AP active agent** | **Polymers** | | **% sweat reduction after 24 hours** |
|---|---|---|---|---|
| | | Name | % AM | |
| **Control** | 15% ACH | Without polymer | | 33% |
| **Example 1:** | 15% ACH | Polymer 1 | 5% | 41% |

The addition of polymer 1 poly(isobornyl acrylate/isobornyl methacrylate/isobutyl acrylate/acrylic acid) makes it possible to significantly increase the antiperspirant efficacy of the aluminium chlorohydrate.
Example 1 has an efficacy of 41%, which is significantly higher than that of the control formulation (33%). The significance threshold p was evaluated in the 2 by 2 mean comparison test according to the Student test. It is considered that the difference between two means is significant when $p < 0.05$. The confidence value p associated with this comparison is 0.0397.
On examining the individual results on 19 volunteers:

- for 9 subjects, the formulation of Example 1 has a perspiration reduction efficacy of 47%; which is higher than for the control example;
- for 7 subjects, the two formulations have an identical perspiration reduction efficacy, which is 37%;
- for 3 subjects, the formulation of Example 1 has a perspiration reduction efficacy of 16%; which is lower than that of the control example.

**Claims**

**1.** Composition in the form of an oil-in-water emulsion comprising, in a cosmetically acceptable medium:

a) a continuous aqueous phase and
b) an oily phase dispersed in the said aqueous phase and
c) at least one water-insoluble block film-forming ethylenic polymer, the said polymer comprising a first block with a glass transition temperature (Tg) of greater than or equal to 85°C and a second block with a Tg of less than or equal to 20°C and
d) at least one aluminium and/or zirconium antiperspirant salt or complex;

wherein said first block with a Tg of greater than or equal to 85°C comprises at least one acrylate monomer of formula $CH_2=CH-COOR$ and at least one methacrylate monomer of formula $CH_2=C(CH_3)-COOR$ in which R, which may be identical or different, represents a $C_4$ to $C_{12}$ cycloalkyl group and preferably a $C_8$ to $C_{12}$ cycloalkyl; and wherein said second block with a Tg of less than or equal to 20°C comprises at least one monomer chosen, alone

or as a mixture, from:

- the acrylates of formula $CH_2=CHCOOR_3$ in which $R_3$ represents a linear or branched $C_1$ to $C_{12}$ unsubstituted alkyl group, with the exception of the tert-butyl group, in which one or more heteroatoms chosen from O, N and S are optionally intercalated;
- the methacrylates of formula $CH_2=C(CH_3)-COOR_4$ in which $R_4$ represents a linear or branched $C_6$ to $C_{12}$ unsubstituted alkyl group, in which one or more heteroatoms chosen from O, N and S are optionally intercalated;
- (meth)acrylic acid; and
- the monomers of formula (I), preferably with x = 1 and Z=COO,

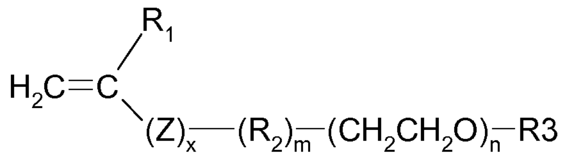

(I)

in which:

- $R_1$ is a hydrogen atom or a methyl radical;
- Z is a divalent group chosen from -COO-, -CONH-, $-CONCH_3-$, -OCO-, -O-, $-SO_2-$, -CO-O-CO- and $-CO-CH_2-CO-$;
- x is 0 or 1;
- $R_2$ is a linear, branched or cyclic, saturated or unsaturated, optionally aromatic divalent carbon-based radical, of 1 to 30 carbon atoms, which may comprise 1 to 18 heteroatoms chosen from O, N, S, F, Si and P;
- m is 0 or 1;
- n is an integer between 3 and 300 inclusive;
- $R_3$ is a hydrogen atom or a linear, branched or cyclic, saturated or unsaturated, optionally aromatic carbon-based radical, of 1 to 30 carbon atoms, which may comprise 1 to 20 heteroatoms chosen from O, N, S, F, Si and P.

**2.** Composition according to Claim 1, in which, in the polymer:

- the block with a Tg of greater than or equal to 85°C represents 50% to 90% by weight and preferably 60% to 80% by weight relative to the weight of the final polymer; and/or
- the block with a Tg of less than or equal to 20°C represents 5% to 50% by weight and preferably 10% to 40% by weight relative to the weight of the final polymer.

**3.** Composition according to either of the preceding claims, in which the said first and second blocks are linked together via an intermediate segment comprising at least one constituent monomer of the said first block and at least one constituent monomer of the said second block.

**4.** Composition according to one of the preceding claims, in which the block ethylenic polymer has a polydispersity index Ip of greater than 2, especially between 2 and 9, preferably between 2.3 and 8 and better still between 2.4 and 7.

**5.** Composition according to one of the preceding claims, in which:

- the weight-average molar mass (Mw) of the block ethylenic polymer is between 35 000 and 300 000 and better still between 45 000 and 150 000 g/mol; and/or
- the number-average molar mass (Mn) of the block ethylenic polymer is between 10 000 and 70 000 and better still between 12 000 and 50 000 g/mol.

**6.** Composition according to one of the preceding claims, in which the block with a Tg of greater than or equal to 85°C comprises at least one monomer with a Tg of greater than or equal to 85°C, chosen from isobornyl (meth)acrylate and isobornyl acrylate.

**7.** Composition according to one of the preceding claims, in which the block with a Tg of less than or equal to 20°C comprises at least one monomer with a Tg of less than or equal to 20°C, chosen from alkyl acrylates in which the alkyl chain comprises from 1 to 10 carbon atoms, with the exception of the tert-butyl group, such as methyl acrylate, isobutyl acrylate and 2-ethylhexyl acrylate; and also poly(ethylene glycol) (meth)acrylates and alkylpoly(ethylene

glycol) (meth)acrylates, more particularly methylpoly(ethylene glycol) methacrylates; and mixtures thereof.

**8.** Composition according to one of the preceding claims, in which the water-insoluble block film-forming ethylenic polymer is chosen from:

- a poly(isobornyl acrylate/isobornyl methacrylate/isobutyl acrylate/acrylic acid) polymer
- an isobornyl acrylate/isobornyl methacrylate/PEG methacrylate/acrylic acid statistical polymer and more particularly a poly(isobornyl acrylate/isobornyl methacrylate/isobutyl acrylate/acrylic acid) polymer.

**9.** Composition according to one of the preceding claims, comprising a combination of:

(A) at least a mixture comprising at least one alkylpolyglycoside whose alkyl chain is linear or branched and comprises from 12 to 22 carbon atoms and at least one linear or branched fatty alcohol containing from 12 to 22 carbon atoms;
(B) at least one associative nonionic polyurethane polyether.

**10.** Composition according to one of the preceding claims, in which the aluminium and/or zirconium salt or complex is aluminium chlorohydrate in activated or non-activated form.

**11.** Composition according to one of the preceding claims, **characterized in that** it is in the form of a cream dispensed in a tube or a grid; in the form of a roll-on (conditioned in ball form) or in pressurized form such as a spray or an aerosol device, and more particularly in roll-on form.

**12.** Cosmetic treatment process for treating human perspiration and, where appropriate, the body odour associated with human perspiration, especially underarm odour, consisting in applying to the surface of the skin to be treated at least one composition as defined according to one of the preceding claims.

## Patentansprüche

**1.** Zusammensetzung in Form einer Öl-in-Wasser-Emulsion, umfassend in einem kosmetisch unbedenklichen Medium:

a) eine kontinuierliche wässrige Phase und
b) eine ölige Phase, die in der wässrigen Phase dispergiert ist, und
c) mindestens ein wasserunlösliches filmbildendes ethylenisches Blockpolymer, wobei das Polymer einen ersten Block mit einer Glasübergangstemperatur (Tg) größer oder gleich 85 °C und einen zweiten Block mit einer Tg kleiner oder gleich 20 °C umfasst, und
d) mindestens ein schweißhemmendes Aluminium- und/oder Zirconiumsalz oder einen schweißhemmenden Aluminium- und/oder Zirconiumkomplex;

wobei der erste Block mit einer Tg größer oder gleich 85 °C mindestens ein Acrylat-Monomer der Formel $CH_2=CH-COOR$ und mindestens ein Methacrylat-Monomer der Formel $CH_2=C(CH_3)-COOR$ umfasst, wobei R, das gleich oder verschieden sein kann, für eine $C_4$- bis $C_{12}$-Cycloalkylgruppe und vorzugsweise ein $C_8$- bis $C_{12}$-Cycloalkyl steht; und
wobei der zweite Block mit einer Tg kleiner oder gleich 20 °C mindestens ein Monomer umfasst, das alleine oder als Mischung aus

- den Acrylaten der Formel $CH_2=CH-COOR_3$, wobei $R_3$ für eine lineare oder verzweigte unsubstituierte $C_1$- bis $C_{12}$-Alkylgruppe mit Ausnahme der tert-Butylgruppe, in die gegebenenfalls ein oder mehrere Heteroatome, die aus O, N und S ausgewählt sind, eingeschoben sind, steht;
- den Methacrylaten der Formel $CH_2=C(CH_3)-COOR_4$, wobei $R_4$ für eine lineare oder verzweigte unsubstituierte $C_6$- bis $C_{12}$-Alkylgruppe, in die gegebenenfalls ein oder mehrere Heteroatome, die aus O, N und S ausgewählt sind, eingeschoben sind, steht;
- (Meth)acrylsäure; und
- den Monomeren der Formel (I), vorzugsweise mit x = 1 und Z = COO,

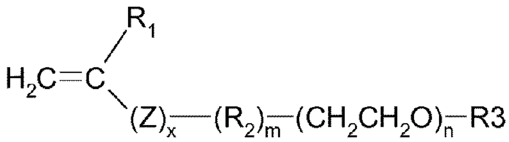

(I)

wobei:

- $R_1$ für ein Wasserstoffatom oder einen Methylrest steht;
- Z für eine zweiwertige Gruppe steht, die aus -COO-, -CONH-, $-CONCH_3$, -OCO-, -O-, $-SO_2-$, -CO-O-CO- und $-CO-CH_2-CO-$ ausgewählt ist;
- x für 0 oder 1 steht;
- $R_2$ für einen linearen, verzweigten oder cyclischen, gesättigten oder ungesättigten, gegebenenfalls aromatischen zweiwertigen Rest auf Kohlenwasserstoffbasis mit 1 bis 30 Kohlenstoffatomen, der 1 bis 18 Heteroatome, die aus O, N, S, F, Si und P ausgewählt sind, umfassen kann, steht;
- m für 0 oder 1 steht;
- n für eine ganze Zahl zwischen 3 und 300 inklusive steht;
- $R_3$ für ein Wasserstoffatom oder einen linearen, verzweigten oder cyclischen, gesättigten oder ungesättigten, gegebenenfalls aromatischen zweiwertigen Rest auf Kohlenwasserstoffbasis mit 1 bis 30 Kohlenstoffatomen, der 1 bis 20 Heteroatome, die aus O, N, S, F, Si und P ausgewählt sind, umfassen kann, steht;

ausgewählt ist.

**2.** Zusammensetzung nach Anspruch 1, wobei in dem Polymer:

- der Block mit einer Tg größer oder gleich 85 °C 50 bis 90 Gew.-% und vorzugsweise 60 bis 80 Gew.-%, bezogen auf das Gewicht des fertigen Polymers, ausmacht; und/oder
- der Block mit einer Tg kleiner oder gleich 20 °C 5 bis 50 Gew.-% und vorzugsweise 10 bis 40 Gew.-%, bezogen auf das Gewicht des fertigen Polymers, ausmacht.

**3.** Zusammensetzung nach einem der vorhergehenden Ansprüche, wobei der erste Block und der zweite Block über ein Zwischensegment, das mindestens ein Aufbaumonomer des ersten Blocks und mindestens ein Aufbaumonomer des zweiten Blocks umfasst, miteinander verknüpft sind.

**4.** Zusammensetzung nach einem der vorhergehenden Ansprüche, wobei das ethylenische Blockpolymer einen Polydispersitätsindex Ip von mehr als 2, speziell zwischen 2 und 9, vorzugsweise zwischen 2,3 und 8 und noch besser zwischen 2,4 und 7 aufweist.

**5.** Zusammensetzung nach einem der vorhergehenden Ansprüche, wobei:

- die gewichtsmittlere Molmasse (Mw) des ethylenischen Blockpolymers zwischen 35.000 und 300.000 und noch besser zwischen 45.000 und 150.000 g/mol liegt; und/oder
- die zahlenmittlere Molmasse (Mn) des ethylenischen Blockpolymers zwischen 10.000 und 70.000 und noch besser zwischen 12.000 und 50.000 g/mol liegt.

**6.** Zusammensetzung nach einem der vorhergehenden Ansprüche, wobei der Block mit einer Tg größer oder gleich 85 °C mindestens ein Monomer mit einer Tg größer oder gleich 85 °C, das aus Isobornyl-(meth)acrylat und Isobornylacrylat ausgewählt ist, umfasst.

**7.** Zusammensetzung nach einem der vorhergehenden Ansprüche, wobei der Block mit einer Tg kleiner oder gleich 20 °C mindestens ein Monomer mit einer Tg kleiner oder gleich 20 °C, das aus Alkylacrylaten, in denen die Alkylkette 1 bis 10 Kohlenstoffatome umfasst, mit Ausnahme der tert-Butylgruppe, wie Methylacrylat, Isobutylacrylat und 2-Ethylhexylacrylat; sowie Poly(ethylenglykol)(meth)acrylaten und Alkylpoly(ethylenglykol)(meth)acrylaten, spezieller Methylpoly-(ethylenglykol)(meth)acrylaten; und Mischungen davon ausgewählt ist, umfasst.

**8.** Zusammensetzung nach einem der vorhergehenden Ansprüche, wobei das wasserunlösliche filmbildende ethylenische Blockpolymer aus:

- einem Poly(isobornylacrylat/isobornylmethacrylat/isobutylacrylat/acrylsäure)-Polymer,
- einem statistischen Isobornylacrylat/Isobornylmethacrylat/PEG-Methacrylat/Acrylsäure-Polymer und spezieller einem Poly(isobornylacrylat/isobornylmethacrylat/isobutylacrylat/acrylsäure)-Polymer ausgewählt ist.

**9.** Zusammensetzung nach einem der vorhergehenden Ansprüche, umfassend eine Kombination von:

(A) mindestens einer Mischung, die mindestens ein Alkylpolyglykosid, dessen Alkylkette linear oder verzweigt ist und 12 bis 22 Kohlenstoffatome umfasst, und mindestens einen linearen oder verzweigten Fettalkohol mit 12 bis 22 Kohlenstoffatomen umfasst;
(B) mindestens einem assoziativen nichtionischen Polyurethanpolyether.

**10.** Zusammensetzung nach einem der vorhergehenden Ansprüche, wobei es sich bei dem Aluminium- und/oder Zirconiumsalz oder -komplex um Aluminiumchlorhydrat in aktivierter oder nicht aktivierter Form handelt.

**11.** Zusammensetzung nach einem der vorhergehenden Ansprüche, **dadurch gekennzeichnet, dass** sie in Form einer in einer Tube oder einem Gitter abgegebenen Creme; in Form eines Rollers (in Ballform konditioniert) oder in druckbeaufschlagter Form wie ein Spray oder eine Aerosolvorrichtung und spezieller in Rollerform vorliegt.

**12.** Kosmetisches Behandlungsverfahren zur Behandlung von menschlicher Perspiration und gegebenenfalls des mit menschlicher Perspiration assoziierten Körpergeruchs, insbesondere Achselgeruch, das darin besteht, dass man auf die Oberfläche der zu behandelnden Haut mindestens eine Zusammensetzung gemäß einem der vorhergehenden Ansprüche aufbringt.

## Revendications

**1.** Composition sous forme d'émulsion huile-dans-eau comprenant, dans un milieu cosmétiquement acceptable :

a) une phase aqueuse continue et
b) une phase huileuse dispersée dans ladite phase aqueuse et
c) au moins un polymère éthylénique filmogène non hydrosoluble et séquencé, ledit polymère comprenant une première séquence présentant une température de transition vitreuse (Tg) supérieure ou égale à 85°C et une deuxième séquence présentant une Tg inférieure ou égale à 20°C et
d) au moins un sel ou complexe anti-transpirant d'aluminium et/ou de zirconium.

où ladite première séquence ayant une Tg supérieure ou égale à 85°C comprend au moins un monomère acrylate de formule $CH_2$=CH-COOR et au moins un monomère méthacrylate de formule $CH_2=C(CH_3)$-COOR dans laquelle R, qui peut être identique ou différent, représente un groupe cycloalkyle en $C_4$ à $C_{12}$ et de préférence un cycloalkyle en $C_8$ à $C_{12}$ ; et ladite deuxième séquence ayant une Tg inférieure ou égale à 20°C comprend au moins un monomère choisi, seul ou en tant que mélange, parmi :

- les acrylates de formule $CH_2=CHCOOR_3$, avec $R_3$ représentant un groupe alkyle en $C_1$ à $C_{12}$ non-substitué linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2=C(CH_3)-COOR_4$, avec $R_4$ représentant un groupe alkyle en $C_6$ à $C_{12}$ non-substitué linéaire ou ramifié, dans lequel se trouve(nt) éventuellement intercalé(s) un ou plusieurs hétéroatomes choisis parmi O, N et S ;
- l'acide (méth)acrylique ; et
- les monomères de formule (I), de préférence avec x = 1 et Z = COO,

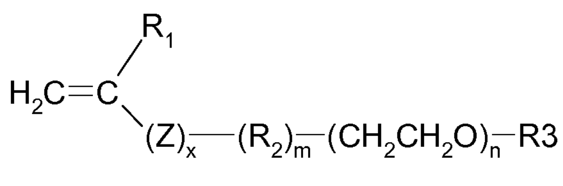

(I)

dans laquelle :

- $R_1$ est un atome d'hydrogène ou un radical méthyle ;
- Z est un groupement divalent choisi parmi -COO-, -CONH-, -$CONCH_3$-, -OCO-, -O-, -$SO_2$- -CO-O-CO- ou -CO-$CH_2$-CO- ;
- x est 0 ou 1 ;
- $R_2$ est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P ;
- m est 0 ou 1 ;
- n est un entier compris entre 3 et 300 inclus ;
- $R_3$ est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P.

**2.** Composition selon la revendication 1, dans laquelle, dans le polymère :

- la séquence de Tg supérieure ou égale à 85°C représente 50 à 90% en poids, de préférence 60 à 80% en poids, du poids du polymère final ; et/ou
- la séquence de Tg inférieure ou égale à 20°C représente 5 à 50% en poids, de préférence 10 à 40% en poids, du poids du polymère final.

**3.** Composition selon l'une des revendications précédentes, dans laquelle lesdites première et deuxième séquences sont reliées entre elles par un segment intermédiaire comprenant au moins un monomère constitutif de ladite première séquence et au moins un monomère constitutif de ladite deuxième séquence.

**4.** Composition selon l'une des revendications précédentes, dans laquelle le polymère éthylénique séquencé a un indice de polydispersité Ip supérieur à 2, notamment compris entre 2 et 9, de préférence entre 2,3 et 8, plus préférablement compris entre 2,4 et 7.

**5.** Composition selon l'une des revendications précédentes, dans laquelle :

- la masse molaire moyenne en poids (Mw) du polymère éthylénique séquencé est comprise entre 35 000 et 300 000, préférablement entre 45 000 et 150 000 g/mol ; et/ou
- la masse molaire moyenne en nombre (Mn) du polymère éthylénique séquencé est comprise entre 10 000 à 70 000, préférablement entre 12 000 et 50 000 g/mol.

**6.** Composition selon l'une des revendications précédentes, dans laquelle la séquence ayant une Tg supérieure ou égale à 85°C, comprend au moins un monomère de Tg supérieure ou égale à 85°C choisi parmi le (méth)acrylate d'isobornyle et l'acrylate d'isobornyle.

**7.** Composition selon l'une des revendications précédentes, dans laquelle la séquence ayant une Tg inférieure ou égale à 20°C, comprend au moins un monomère de Tg inférieure ou égale à 20°C choisi parmi les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2-hexyle ; ainsi que les (méth)acrylates de poly(éthylène glycol) et les (méth)acrylates d'alkyl-poly(éthylène glycol), plus particulièrement les méthacrylates de méthyl-poly(éthylène glycol) ; et leurs mélanges.

**8.** Composition selon l'une des revendications précédentes, dans laquelle le polymère éthylénique filmogène non hydrosoluble et séquencé est choisi parmi :

- un polymère de poly(acrylate d'isobornyle /méthacrylate d'isobornyle /acrylate d'isobutyle/acide acrylique)
- un polymère statistique acrylate d'isobornyle/méthacrylate d'isobornyle/méthacrylate de PEG/ acide acrylique et plus particulièrement un polymère de poly(acrylate d'isobornyle /méthacrylate d'isobornyle /acrylate d'isobutyle/acide acrylique).

**9.** Composition selon l'une des revendications précédentes, comprenant l'association de :

(A) au moins un mélange comprenant au moins un alkylpolyglycoside dont la chaîne alkyle est linéaire ou ramifiée et comprend de 12 à 22 atomes de carbone et au moins un alcool gras, linéaire ou ramifié, ayant de 12 à 22 atomes de carbone ;

(B) au moins un polyéther polyuréthane non-ionique associatif.

**10.** Composition selon l'une des revendications précédentes, dans laquelle le sel ou complexe d'aluminium et/ou de zirconium est le chlorhydrate d'aluminium sous forme activée ou non.

**11.** Composition selon l'une des revendications précédentes, **caractérisée par le fait qu'**elle se présente sous forme de crème distribuée en tube ou en grille ; sous forme de roll-on (conditionnée sous forme de bille) ou sous forme pressurisée comme un spray ou un dispositif aérosol et plus particulèrement sous forme de roll-on.

**12.** Procédé de traitement cosmétique pour traiter la transpiration humaine et éventuellement les odeurs corporelles liées à la transpiration humaine, notamment les odeurs axillaires consistant à appliquer sur la surface de la peau à traiter au moins une composition telle que définie selon l'une des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9324105 A
- WO 9527473 A
- WO 0154658 A
- US 6387356 B
- DE 2947060
- WO 2011073356 A
- FR 2954150
- EP 8424911 A
- WO 9623483 A
- US 5919441 A
- WO 2005030155 A
- EP 1374843 A
- EP 1103249 A
- US 3792068 A
- WO 9206778 A
- WO 9513863 A
- WO 9847610 A
- DE 102008012457
- EP 847752 A
- FR 2792190 A
- WO 02056847 A
- WO 0247619 A
- US 5783657 A
- US 5874069 A
- US 6051216 A
- US 5981680 A
- US 5002698 A

### Non-patent literature cited in the description

- **BRANDRUP ; IMMERGUT ; GRULKE.** Polymer Handbook. John Wiley, 1999
- **G. FONNUM ; J. BAKKE ; FK. HANSEN.** *Colloid Polym. Sci.,* 1993, vol. 271, 380-389
- CTFA. 2000